# EUROPEAN PATENT SPECIFICATION

(11) **EP 2 937 090 B1**
(45) Date of publication and mention of the grant of the patent: **10.06.2020**
(21) Application number: 13866361.2
(22) Date of filing: 20.12.2013
(51) Int. Cl.: A61K 35/407, A61K 35/12, A61K 38/00, A61K 38/17, A61K 38/43, A61P 43/00, C12M 1/00

(54) **TISSUE REGENERATION PROMOTING AGENT**
GEWEBEREGENERATIONSFÖRDERER
AGENT PROMOTEUR DE RÉGÉNÉRATION TISSULAIRE

(30) Priority: 21.12.2012 JP 2012280261
(43) Date of publication of application: 28.10.2015
(73) Proprietor: Nitto Denko Corporation, Ibaraki-shi, Osaka 567-8680 (JP)
(72) Inventor: NIITSU, Yoshiro, Sapporo-shi Hokkaido 064-0823 (JP); YONEDA, Akihiro, Sapporo-shi, Hokkaido 0600005 (JP)
(74) Representative: Hoffmann Eitle
(86) International application number: PCT/JP2013/084228
(87) International publication number: WO 2014/098211

(56) References cited:
- WO-A2-2004/019921
- WO-A2-2010/079340
- WO-A2-2011/109767
- WO-A2-2012/064834
- WO-A2-2012/129517
- JP-A- 2008 054 276
- US-A1- 2007 015 242
- YOSHIRO NIITSU ET AL.: 'Bunshi Saibo kara Kaimei suru Kanzo Bioloqy Kansei Saibo o Hyoteki to shita Kankohen no Chiryo Senryaku' EXPERIMENTAL MEDICINE vol. 29, no. 13, 2011, pages 2114 - 2122, XP008172815
- 'Hyoteki to shita Kankohen no Chiryo Senryaku' EXPERIMENTAL MEDICINE vol. 29, no. 13, 2011, pages 2114 - 2122, XP008172815
- KEIKO ASAI ET AL: "Activated hepatic stellate cells overexpress p75NTR after partial hepatectomy and undergo apoptosis on nerve growth factor stimulation", LIVER INTERNATIONAL, vol. 26, no. 5, 1 June 2006 (2006-06-01), pages 595-603, XP55506401, GB ISSN: 1478-3223, DOI: 10.1111/j.1478-3231.2006.01267.x
- DUNCAN A W ET AL: "Stem Cells and Liver Regeneration", GASTROENTEROLOGY, W.B. SAUNDERS CO, US, vol. 137, no. 2, 1 August 2009 (2009-08-01), pages 466-481, XP026374514, ISSN: 0016-5085 [retrieved on 2009-05-24]
- DAVIS ET AL: "Affinity of integrins for damaged extracellular matrix: @a"v@b"3 binds to denatured collagen type I through RGD sites", BIOCHEMICAL AND BIOPHYSICAL RESEARCH COMMUNICATIONS, ELSEVIER, AMSTERDAM, NL, vol. 182, no. 3, 14 February 1992 (1992-02-14), pages 1025-1031, XP024838221, ISSN: 0006-291X, DOI: 10.1016/0006-291X(92)91834-D [retrieved on 1992-02-14]

## Description

### [Technical Field]

The present invention relates to a composition for use in and methods for promoting tissue regeneration, promoting cell differentiation and/or promoting cell proliferation, comprising a collagen that has been treated with MMP14, as defined by the appended claims.

### [Background Art]

When a tissue of living organisms is damaged, it regenerates so as to compensate for the damaged portion, and attempts to restore its function. For example, it is known that when more than half of a liver is removed, the liver can regenerate into almost its original size and restore its function within a short period of time. Studies on tissue regeneration have long been conducted centering on the liver, and various findings have been reported; however, detailed mechanism of tissue regeneration has not yet been clarified. Multiple types of cells are considered to be involved in a complex manner in tissue regeneration; however, even for which one of these cells play a major role, no definitive conclusion has been obtained.

Elucidation of a mechanism of tissue regeneration has been conducted by analyzing time course changes in damaged tissues using tissue staining and cell staining, as well as effects of various physiologically active substances on tissue regeneration. For example, in Non-patent Document 1, the following hypothesis of liver regeneration is shown using a mouse partial hepatectomy model: hepatic sinusoidal endothelial cells of a particular phenotype induce proliferation of liver cells by up-regulation of Id1, a endothelial cell-specific transcription factor, via vascular endothelial growth factor-A receptor-2 (VEGFR2), which is followed by proliferation of the endothelial cells themselves, leading to liver regeneration.

However, despite the strenuous studies, only a small portion of the mechanism of tissue regeneration has been clarified and further research efforts are required.

### [Citation List]

### Non-patent Document

Non-patent Document 1: Ding et al., Nature. 2010 Nov. 11; 468 (7321): 310-5

WO 2004/019921 relates to the treatment of liver disease. WO 2011/109767 relates to gelatinase inhibitors and prodrugs. WO 2012/129517 relates to the evaluation of protein expression in patient stratification and other therapeutic, diagnostic and prognostic methods for cancer. WO 2010/079340 relates to a method for identifying compounds that act as enhancers or inhibitors or matrix metalloproteinase (MMP) activity. US 2007/015242 relates to fragments of type XVIII collagen termed neostatins. WO 2012/064834 relates to methods for organ regeneration. Asai et al., Liver Int. 2006 Jun;26(5):595-603, report that activated hepatic stellate cells overexpress p75NTR after partial hepatectomy and undergo apoptosis on nerve growth factor stimulation. Duncan et al., Gastroenterology. 2009 Aug;137(2):466-81, discuss stem cells and liver regeneration. Zhou et al., Liver Int. 2004 Oct;24(5):492-501, report that expression of matrix metal loproteinase-2 and -14 persists during early resolution of experimental liver fibrosis and might contribute to fibrolysis. Davis, Biochem Biophys Res Commun. 1992 Feb 14; 182(3): 1025-31, reports on the affinity of integrins for damaged extracellular matrix, and that alpha v beta 3 binds to denatured collagen type I through RGD sites.

### [Disclosure of Invention]

### [Problems to Be Solved by the Invention]

An object of the present invention is to provide a novel tissue regeneration promoting agent, and a method for promoting tissue regeneration.

### [Means for Solving the Problems]

The present inventors have found the following in the course of extensive research to solve the above problems: 1) activated stellate cells play an important role in the regeneration of tissue; 2) secretion products of activated stellate cells induce proliferation and differentiation of stem cells that are the core of tissue regeneration; 3) collagen that has been subjected to the action of MMP14 expressed by the activated stellate cells induces proliferation of parenchymal cells of the tissue; and 4) RGD sequence present in the collagen is involved in the induction of this proliferation; thus, the inventors have completed the present invention.

Namely, the present invention is as defined by the appended claims.

### [Advantageous Effects of the Invention]

According to the present invention, not only in vivo tissue regeneration, but also in vitro tissue formation can be promoted; therefore, significant contribution in the biological and medical fields can be expected.

Moreover, the promotion of tissue regeneration according to the invention is particularly useful in situations wherein tissue regeneration is suppressed, for example, a situation involving a disease such as fibrosis.

### [Brief Description of Drawings]

[Fig. 1] Figure 1 is a diagram showing the flow of treatment of partially hepatectomized rats.
[Fig. 2] Figure 2 is a photograph showing appearance of livers of each group collected on day 5 and day 10 after partial hepatectomy, in comparison to the liver before and immediately after the partial hepatectomy.
[Fig. 3] Figure 3 is a graph showing change in the liver weight of livers collected in each group.
[Fig. 4] Figure 4 is a diagram showing fluorescence microscope images of the liver tissue collected on day 5 after partial hepatectomy and co-stained with FITC-TUNEL, α-SMA and DAPI.
[Fig. 5] Figure 5 is a graph showing the number of α-SMA-positive TUNEL-positive cells in the fluorescence microscope images of the liver tissue collected on day 5 after partial hepatectomy and co-stained with FITC-TUNEL, α-SMA and DAPI.
[Fig. 6] Figure 6 is a diagram showing fluorescence microscope images of the liver tissue collected on day 5 after partial hepatectomy and co-stained with Ki67 and DAPI.
[Fig. 7] Figure 7 is a graph showing the number of Ki67-positive cells in the fluorescence microscope images of the liver tissue collected on day 5 after partial hepatectomy and co-stained with Ki67 and DAPI.
[Fig. 8] Figure 8 is a diagram showing fluorescence microscope images of the liver tissue collected on day 5 after partial hepatectomy and co-stained with CD133 and DAPI.
[Fig. 9] Figure 9 is a diagram showing outline of the treatment applied to each group of Example 3.
[Fig. 10] Figure 10 is a diagram showing the appearance of livers collected in each group, in comparison to the liver immediately after partial hepatectomy (day 0).
[Fig. 11] Figure 11 is a graph showing the weight of livers collected in each group, in comparison to that of the liver immediately after partial hepatectomy (day 0).
[Fig. 12] Figure 12 is a diagram showing time course change in α-SMA-positive cells of the liver tissue after partial hepatectomy.
[Fig. 13] Figure 13 is a graph showing time course change in the number of α-SMA-positive cells of the liver tissue after partial hepatectomy.
[Fig. 14] Figure 14 is a diagram showing microscope images of quiescent hepatic stellate cells (top view) and activated hepatic stellate cells (bottom view).
[Fig. 15] Figure 15 is a diagram showing microscope images of GFP-positive/albumin-positive colonies in the contact co-culture of stem cells and stellate cells.
[Fig. 16] Figure 16 is a diagram showing microscope images of GFP-positive/albumin-positive colonies in contact co-culture of stem cells and stellate cells, when EGF and HGF are added.
[Fig. 17] Figure 17 is a graph showing the number of GFP-positive/albumin-positive colonies in contact co-culture of stem cells and stellate cells.
[Fig. 18] Figure 18 is a graph showing the area of GFP-positive/albumin-positive colonies in contact co-culture of stem cells and stellate cells.
[Fig. 19] Figure 19 is a graph showing the area of GFP-positive/albumin-positive colonies in non-contact co-culture of stem cells and stellate cells.
[Fig. 20] Figure 20 is a graph showing proliferation of GFP-positive/albumin-positive cells in non-contact co-culture of stem cells and stellate cells, represented in terms of absorbance.
[Fig. 21] Figure 21 shows microscope images showing time course change in the DNA synthesis in liver tissue after partial hepatectomy.
[Fig. 22] Figure 22 is a graph showing time course change in the percentage of BrdU-positive cells in hepatic cell after partial hepatectomy.
[Fig. 23] Figure 23 shows microscope images showing time course change in the DNA synthesis in liver tissue after partial hepatectomy.
[Fig. 24] Figure 24 is a graph showing time course change in the percentage of BrdU-positive cells in liver tissue after partial hepatectomy.
[Fig. 25] Figure 25 shows microscope images showing BrdU-positive liver cells when stem cells and stellate cells are co-cultured.
[Fig. 26] Figure 26 is a graph showing the ratio of BrdU-positive liver cells, when stem cells and stellate cells are co-cultured.
[Fig. 27] Figure 27 shows microscope images showing BrdU-positive cells, when liver cells are cultured on collagen that has been subjected to various treatments.
[Fig. 28] Figure 28 is a graph showing the ratio of BrdU-positive cells, when liver cells are cultured on collagen that has been subjected to various treatments.
[Fig. 29] Figure 29 shows microscope images showing BrdU-positive cells, when hepatocytes and MMP14- and/or HGF-knocked down stellate cells are co-cultured.
[Fig. 30] Figure 30 is a graph showing the ratio of BrdU-positive cells, when liver cells and MMP14- and/or HGF-knocked down stellate cells are co-cultured.
[Fig. 31] Figure 31 shows microscope images showing BrdU-positive cells, when liver cells are cultured on collagen that has been treated with MMP14 under the presence of RGD peptide.
[Fig. 32] Figure 32 is a graph showing the ratio of BrdU-positive cells, when hepatocytes are cultured on collagen that has been treated with MMP14 under the presence of RGD peptide.

### [Description of Embodiments]

One aspect of the present invention relates to a composition for use in promoting tissue regeneration, comprising a collagen that has been treated with MMP14, as defined by the appended claims 1-6.

Activated stellate cells can be obtained by subculturing stellate cells isolated from a living body. Stellate cells are known to be present in various tissues such as liver, pancreas, kidney, intestine and lung (Zhao and Burt, J Mol Histol. 2007 Mar; 38 (1) : 53-64), and any of these may be used. Stellate cells can be isolated using any known method. Specific examples of a method for isolating hepatic stellate cells are illustrated in Example 5 (1) below. Activated stellate cells are characterized by expression of αSMA, and they can be selected using αSMA as a marker .

Activated stellate cells may be those which have been subjected to a treatment for increasing the expression of a protein, selected from the group consisting of HGF, EGF, and MMP14 . Examples of such treatment include, but are not limited to, introduction of a gene encoding said protein in the activated stellate cells. Genes encoding HGF, EGF and MMP14 are known, and a method for introducing genes are well-known in the art.

A decomposition product of activated stellate cells can be obtained by decomposing activated stellate cells using a variety of techniques including physical and/or chemical methods. Decomposition can be carried out by any method known to decompose cells, for example, osmotic shock method, freezing and thawing method, the use of surfactants, enzymatic digestion, sonication, French press, and crushing with a mortar, crushing by a homogenizer, and crushing by glass beads. Decomposition technique without denaturing proteins or with a slight degree of denaturation is preferred. By using such a technique, a MMP14 which is expressed on the cell membrane can be obtained without impairing its function. Furthermore, decomposition products of activated stellate cells preferably comprise a cell membrane component.

MMP14 (also referred to as MT1-MMP) is a MMP expressed on the cell membrane. MMP14 can act on collagen I. The present inventors have revealed that the effect of MMP14 on collagen I is deeply involved in cell proliferation.

MMP14 in the present invention includes not only those expressed on the cell membrane, but also those released from the cell membrane. MMP14 may also be those naturally occurring, or may be those that are artificially produced. Therefore, MMP14 includes recombinant MMP14. MMP14 of free form is known (for example, Jo et al., Biochem J. 2000 Feb 1; 345 Pt 3: 511-9), and MMP14 is also commercially available (for example, R&D Systems, Cat. No. 918-MP-010, 918-MPN-010, etc.) . Amino acids and the base sequence encoding the same for MMP14 are known (for example, the base sequence of human MMP14 is registered as GenBank Accession No. NM_004995, and the amino acid sequence is registered as GenBank Accession No. NP_004986).

MMP14 in the present invention also includes a functional variant thereof. Functional variants of MMP14 include, but are not limited to, the following: (i) a variant having one or more, typically one or several mutations in the amino acid sequence of said protein, and still having equivalent functions of said protein, (ii) a variant encoded by a nucleic acid having one or more, typically one or several mutations in the base sequence of the nucleic acid that has the base sequence of the gene encoding said protein, or that encodes the same polypeptide as this nucleic acid, and having equivalent functions, (iii) a variant encoded by a nucleic acid that hybridizes the complementary strand, or a fragment thereof, of the nucleic acid that has the base sequence of the gene encoding said protein, or that encodes the same polypeptide as this nucleic acid, or that encodes the variant of (ii), under stringent conditions, and having equivalent functions of said protein, (iv) a variant having an amino acid sequence that has 60% or more, preferably 70% or more, more preferably 80% or more, still more preferably 90% or more, and particularly preferably 95% or more homology to the amino acid sequence of said protein, and having equivalent functions of said protein, (v) a variant encoded by a nucleic acid that has 60% or more, preferably 70% or more, more preferably 80% or more, still more preferably 90% or more, and particularly preferably 95% or more homology to the base sequence of the gene encoding said protein, and having equivalent functions of said protein, and the like.

Those skilled in the art can appropriately produce the above functional variants based on the sequence information of MMP14, using any known techniques, such as chemical synthesis, cleavage or insertion of nucleic acid by restriction enzyme, site-specific mutagenesis, irradiation or ultraviolet irradiation.

Whether or not a certain variant has equivalent functions as those of MMP14 can be evaluated by analyzing said variant in terms of known functions of MMP14, for example, without limitation, collagen degradation ability, using any known method, and by comparing it with an appropriate negative control and MMP14 as a positive control. For example, in one variant, if the above function is better than the negative control, for example, 10% or more, 25% or more, 50% or more, 75% or more, and even 100% or more better than that of the negative control, and/or, if this function is 1/100 or more, 1/50 or more, 1/25 or more, 1/10 or more, 1/5 or more, and even 1/2 or more of that of CSABP, then this variant is included in the functional variant of MMP14.

The term "stringent conditions" as used herein refers to well-known parameters in the art, which are described in standard protocols such as Sambrook et al. , Molecular Cloning: A Laboratory Manual, 3d ed., Cold Spring Harbor Press (2001) ; Ausubel et al. , Current Protocols in Molecular Biology, Greene Publishing Associates (1992); and others.

Stringent conditions in the present invention refer to, for example, hybridization at 65°C using a hybridization buffer consisting of 3.5 x SSC (0.15 M sodium chloride/0.15 M sodium citrate, pH 7), 0.02% Ficoll, 0.02% polyvinyl pyrrolidone, 0.02% bovine serum albumin, 25 mM NaH₂PO₄ (pH 7), 0.05% SDS, and 2 mM EDTA. After hybridization, the membrane to which DNA has been transferred is washed with 2 x SSC at room temperature, then with 0.1-0.5 x SSC/0.1 x SDS up to the temperature of 68°C. Alternatively, stringent hybridization may be performed using a commercially available hybridization buffer, such as ExpressHyb(R) Hybridization Solution (Clontech Laboratories, Inc.) under hybridization and washing conditions described by the manufacturer.

Other available conditions and reagents which result in comparable stringency exist; because those skilled in the art are considered to be familiar with such conditions, these are not specifically described herein. However, it is possible to manipulate the conditions in order to enable clear identification of nucleic acids encoding protein variants.

MMP14 and/or a functional variant thereof of the present invention include, in addition to said protein itself or a functional variant thereof, a nucleic acid encoding said protein or a functional variant thereof.

Collagen treated with MMP14 can be obtained by treating collagen with MMP14. As the collagen, for example, collagen I may be used. MMP14 used for the treatment may be, in addition to the above MMP14 (including functional variants of MMP14), the cells themselves which express MMP14, or a decomposition product of the cells which comprises MMP14 . Treatment time by MMP14 may be, for example, at a temperature at which MMP14 can act, 1-120 h, 3-60 h, 6-48 h, and 12-36 h, etc. Collagen treated with MMP14 is in a condition wherein RGD sequence is ready to interact with cells.

A secretion product of activated stellate cells can be obtained from a culture supernatant of activated stellate cells. A culture supernatant may be used as it is, or may be used after concentration by dialysis or freeze drying, etc. Since secretion products of activated stellate cells contain a variety of proteins, preferably, they are handled such that the proteins are not denatured.

Tissue for which regeneration is promoted by the composition of the present invention is liver.

The composition of the present invention is useful for tissue regeneration that occurs in damaged tissues or transplanted tissues. Damaged tissues include those subjected to tissue destruction, inflammation, necrosis, fibrosis, surgical invasion, and organ failure, etc. Administration time of the composition of the present invention is not particularly limited, and preferably the composition is administered within four days from the day of receiving damage or transplantation, or within one day from the day of receiving damage or transplantation.

Tissue regeneration by the composition of the present invention may involve differentiation and/or proliferation of stem cells. Also, tissue regeneration by the composition of the present invention may involve proliferation of the tissue parenchymal cells. Differentiation of stem cells can be evaluated by, for example, detection of cellular markers specific for differentiated cells, and detection of functions exhibited by differentiated cells. Cell proliferation can be evaluated by various known techniques, for example, measurement of living-cell count over time, size, volume or weight of the tissue, measurement of DNA synthesis level, WST-1 method, BrdU (bromodeoxyuridine) method, ³H-thymidine incorporation method and others.

The composition of the present invention may be used for a subject having a condition in which tissue regeneration is suppressed. The condition in which tissue regeneration is suppressed includes, without limitation, for example, inflammation, necrosis, fibrosis, organ failure, decreased platelet count, genetic abnormalities, and reduction of noradrenaline.

The amount of an active ingredient in the composition of the present invention may be an amount with which tissue regeneration is promoted upon administration of the composition. In addition, the amount that does not cause an adverse effect exceeding the benefits of administration is preferred. Such an amount is either publicly known, or may be appropriately determined by an in vitro test using cultured cells, and by a test in a model animal such as mouse, rat, dog or pig, and such test methods are well-known to those skilled in the art. Promotion of tissue regeneration can be evaluated by the recovery of functions, weight and size of the tissue, by means of biochemical tests and image diagnosis using X-ray, ultrasound, MRI, CT, and endoscopy. The amount of an active ingredient may vary according to dosage form of the composition. For example, when multiple units of a composition are used for single administration, the amount of an active ingredient to be blended into one unit of the composition may be the amount of the active ingredient required for the single administration divided by the multiple times . Such amount can be appropriately adjusted by those skilled in the art.

The present disclosure also relates to a method for producing a composition to promote tissue regeneration, comprising blending a component selected from the group consisting of activated stellate cells, a decomposition product of activated stellate cells, MMP14, collagen that has been treated with MMP14, and a secretion product of activated stellate cells; and to the use of said component in the production of a composition for promoting tissue regeneration, and to said component used for promoting tissue regeneration.

Each component and the amount thereof in the above production method or use are as already described above . Blending of each component can be carried out according to any known technique.

The present disclosure also relates to a method for promoting tissue regeneration in a subject, comprising a step of administering the above composition to the subject in need thereof . The subject in the present method may be either those having damaged tissue or tissue transplantation. Damages include, without limitation, for example, tissue destruction, inflammation, necrosis, fibrosis, surgical invasion, organ failure and the like. Administration of the composition may be, for example, within four days from the day of receiving damage or transplantation, or within one day from the day of receiving damage or transplantation.

The present invention also relates to a composition for use in differentiation and/or proliferation of stem cells as defined in the appended claim 7, comprising a collagen that has been treated with MMP14; and a method for defferentiation and/or proliferation of stem cells, as defined in the appended claim 8.

Stem cells are not particularly limited, and include, for example, tissue stem cells (somatic stem cells, adult stem cells), embryonic stem cells, iPS cells and the like. Stem cells may be totipotent, pluripotent, multipotent or unipotent. Examples of tissue stem cells include, but are not limited to, neural stem cells, hematopoietic stem cells, mesenchymal stem cells, liver stem cells, pancreatic stem cells, skin stem cells, muscle stem cells, germ stem cells, etc. Stem cells may be of autologous, or of other individuals of the same species or individuals of different species. Stem cells that have been differentiated and/or proliferated can be transplanted into a subject in need thereof.

The above method is performed in vitro. Each component and an amount thereof in the above composition, method or use are as already described above. Blending of the components can be carried out according to any known technique. When MMP14 is used as an active ingredient in the above composition, method or use, it is preferred that collagen (especially collagen I) is present around the cells of interest in promoting proliferation.

The present invention also relates to a composition for use in promoting cell proliferation as defined in the appended claim 9, comprising a collagen that has been treated with MMP14; and a method for promoting cell proliferation as defined in the appended claim 10.

Cells the proliferation of which is promoted are cells of the liver. Cells may be of autologous, or of other individuals of the same species or individuals of different species. Cells that have been differentiated and/or proliferated can be transplanted into a subject in need thereof.

The above method is performed in vitro. Each component and an amount thereof in the above composition, method or use are as already described above. Blending of the components can be carried out according to any known technique. When MMP14 is used as an active ingredient in the above composition, method or use, it is preferred that collagen (especially collagen I) is present around the cells of interest in promoting proliferation.

The present disclosure also relates to a composition for suppressing cell proliferation, comprising a substance that suppresses MMP14; a method for producing a composition for suppressing cell proliferation, comprising blending said substance; a use of said substance in the production of a composition for suppressing cell proliferation; said substance used for suppressing cell proliferation; and a method for suppressing cell proliferation, comprising a step of contacting said substance with the cells.

The present disclosure also relates to a composition for treating cellular proliferative disease, comprising a substance that suppresses MMP14; a method for producing a composition for treating cellular proliferative disease, comprising blending said substance; a use of said substance in the production of a composition for treating cellular proliferative disease; said substance used for treating cellular proliferative disease; and a method for treating cellular proliferative disease, comprising administrating a therapeutically effective amount of said substance to a subject in need thereof.

Examples of a substance that inhibits MMP14 include, without limitation, a drug that inhibits production and/or activity of MMP14, and a drug that promotes decomposition and/or inactivation of MMP14. Examples of a drug that inhibits production of MMP14 include, without limitation, RNAi molecule for DNA encoding MMP14, ribozyme, antisense nucleic acid, DNA/RNA chimera polynucleotide, and vectors expressing thereof.

Inhibition of MMP14 can be determined by a degree of inhibition of expression or activity of MMP14 in cells, compared to the case wherein an MMP14 inhibitor is not reacted. Expression of MMP14 can be evaluated by any known techniques including, without limitation, for example, immunoprecipitation method utilizing an anti-MMP14 antibody, EIA, ELISA, IRA, IRMA, Western blotting, immunohistochemistry, immunocytochemistry, flow cytometry, various hybridization methods, Northern blotting , Southern blotting, and a variety of PCR methods, utilizing a nucleic acid that specifically hybridizes to the nucleic acid encoding MMP14 or an unique fragment thereof, or to a transcription product (e.g., mRNA) or a splicing product of said nucleic acid.

As used herein, RNAi molecules refer to any molecules that provide RNA interference, and examples include, without limitation, double-stranded RNA such as small interfering RNA (siRNA), micro RNA (miRNA), short hairpin RNA (shRNA), DNA-directed RNA (ddRNA), Piwi-interacting RNA (piRNA), repeat associated siRNA (rasiRNA), and a variant thereof. These RNAi molecules are commercially available, or they can be designed and produced based on known sequence information, etc.

Furthermore, an antisense nucleic acid as used herein includes RNA, DNA, PNA, or a composite thereof.

As used herein, DNA/RNA chimera polynucleotide is not limited, and includes, for example, a double-stranded polynucleotide composed of DNA and RNA that inhibits expression of a target gene, as described in JP 2003-219893.

Examples of cells the proliferation of which is suppressed include, without limitation, cells the proliferation of which has adverse effects, and cells the proliferation of which is involved in diseases. Specifically, they include tumor cells, cancer cells, activated stellate cells and the like. A substance that suppresses MMP14 may be administered to these cells, and it may also be administered to MMP14-expressing cells that support the proliferation of these cells, for example, cancer-associated fibroblast (CAF).

Examples of cellular proliferative disease include, without limitation, benign or malignant tumors, hyperplasia, keloid, Cushing syndrome, primary aldosteronism, erythroplakia, polycythemia vera, leukoplakia, hyperplastic scar, lichen planus and lentiginosis.

When the active ingredient of various compositions and methods described herein is a nucleic acid, such as RNAi molecule, ribozyme, antisense nucleic acid, and DNA/RNA chimera polynucleotide, these may be used as a bare nucleic acid as it is, or these may be supported by various vectors . As the vector, any publicly known vectors such as plasmid vectors, phage vectors, phagemid vectors, cosmid vectors, and viral vectors can be used. Preferably, the vector comprises at least a promoter that enhances the expression of a supporting nucleic acid; in this case, the nucleic acid is preferably operably linked to such promoters. The phrase "nucleic acid is operably linked to promoter" means that the nucleic acid and the promoter are located such that the protein encoded by the nucleic acid can be suitably produced by the action of the promoter. The vector may or may not be capable of replication in a host cell, and transcription of genes may take place outside the nucleus of the host cell, or may take place in the nucleus. In the latter case, the nucleic acid may be incorporated into the genome of the host cell.

Furthermore, the active ingredient can be supported on a variety of non-viral lipids or protein carriers . Examples of such carriers include, without limitation, cholesterol, liposomes, antibody protomers, cyclodextrin nanoparticles, fusion peptides, aptamers, biodegradable polylactic acid copolymers, polymers and the like, and they can enhance the incorporation efficiency into cells (for example, see Pirollo and Chang, Cancer Res. 2008; 68 (5): 1247-50, etc.). In particular, cationic liposomes and polymers (such as polyethyleneimine) are useful. Further examples of useful polymers as such carriers include those described in US 2008/0207553, US 2008/0312174 and the like.

Various compositions of the invention as described herein can be used in medical applications such as in vivo tissue regeneration and treatment of diseases. Thus, various compositions of the present invention can be pharmaceutical compositions. In a pharmaceutical composition, as long as effectiveness of the active ingredient is not interfered, the active ingredient may be combined with other optional ingredients. Such optional ingredients include, for example, other chemotherapeutic agents, pharmaceutically acceptable carriers, excipients, and diluents, etc. Also, depending on the administration route and the drug release manner, the composition may be coated with a suitable material, for example, enteric coating and timed-disintegrating material, and the composition may be incorporated into appropriate drug release systems.

Various compositions of the invention as described herein (including various pharmaceutical compositions) may be administered via various routes including both oral and parenteral routes, for example, without limitation, oral, intravenous, intramuscular, subcutaneous, topical, intratumoral, rectal, intraarterial, intraportal, intraventricular, transmucosal, transdermal, intranasal, intraperitoneal, intrapulmonary, and intrauterine routes, and they may be formulated in a dosage form suitable for each administration route. Such dosage forms and formulation methods may be selected as appropriate from any known ones (see, for example, "Hyojun Yakuzaigaku" (Standard Pharmaceutical Science), Ed. by Yoshiteru Watanabe, et al., Nankodo, 2003).

Examples of dosage forms suitable for oral administration include, but are not limited to, powders, granules, tablets, capsules, solutions, suspensions, emulsions, gels, and syrups, and examples of dosage forms suitable for parenteral administration include injections such as an injectable solution, an injectable suspension, an injectable emulsion, and an injection in a form that is prepared at the time of use. Formulations for parenteral administration may be in the form of transplantation, or aqueous or nonaqueous isotonic sterile solutions or suspensions.

Various compositions of the invention as described herein (including various pharmaceutical compositions) may be targeted to specific tissues and cells. Targeting can be accomplished by any known technique. When delivery to cancer is contemplated, the following techniques can be used without limitation: passive targeting, in which a formulation is made to have a size suitable for exhibiting enhanced permeability and retention (EPR) effect, such as a diameter of 50-200 µm, in particular 75-150 µm; and active targeting, in which a ligand such as CD19, HER2, transferrin receptor, folate receptor, VIP receptor, EGFR (Torchilin, AAPS J. 2007; 9 (2) : E128-47), RAAG10 (JP A 2005-532050), PIPA (JP A 2006-506071), KID3 (JP A 2007-529197), etc., and a peptide having RGD motif and NGR motif, and F3, LyP-1 (Ruoslahti et al., J Cell Biol. 2010; 188(6) :759-68) are used as a targeting agent. Moreover, since retinoid or a derivative thereof is also known to be useful as a targeting agent for cancer cells and CAF (WO 2008/120815), it is also possible to utilize a carrier comprising retinoid as a targeting agent. Such carriers are described, in addition to the above literatures, in WO 2009/036368, WO 2010/014117, and WO 2012/170952.

Various compositions of the invention as described herein (including various pharmaceutical compositions) may be supplied in any form; however, from the viewpoint of storage stability, they may be supplied in a form that can be prepared at the time of use, for example, in a form that can be prepared at the site of clinical practice or in the vicinity thereof by a physician and/or pharmacist, nurse or other paramedical personnel, etc. Such a form is particularly useful when the composition of the present invention comprises a component that is difficult to store in a stable manner, such as lipids, proteins, and nucleic acids. In this case, the composition of the present invention is provided in one or more containers which further comprise at least one of essential constituents, and the composition is prepared, for example, within 24 h, preferably 3 h prior to use, and more preferably immediately before use. Upon preparation, reagents, solvents and dispensing equipment usually available in a place of preparation can be used as appropriate.

Accordingly, the present disclosure also relates to a kit for preparation of a composition, comprising one or more containers containing singly or in combination active ingredients which may be included in various compositions of the present invention; and also relates to a constituent element necessary for various compositions provided in a form of such a kit. The kit may comprise, in addition to the above, instructions describing preparation method and administration method of the various compositions disclosure herein, for example, an instruction, or an electronic recording medium such as CD, DVD, etc. Furthermore, the kit may include all of the constituent elements for completing the various compositions of the invention, but need not always include all of the constituent elements. Therefore, the kit may not include a reagent or a solvent usually available at the site of clinical practice and experimental facility, such as sterile water, physiological saline, and glucose solution, etc.

An effective amount in the various methods of the invention described herein may be, for example, with respect to regeneration of tissues, an amount that promotes tissue regeneration, or eliminates delay in tissue regeneration; with respect to treatment of a disease, an amount that reduces symptoms of the disease, or delays or stops progression of the disease, and preferably, an amount that suppresses or cures the disease. In addition, an amount that does not cause an adverse effect exceeding the benefits from administration is preferred. Such an amount can be appropriately determined by an in vitro test using cultured cells, or a test in a model animal such as mouse, rat, dog or pig, and such test methods are well known to those skilled in the art. Furthermore, dosage of a drug used in the treatment methods of the invention is either known to those skilled in the art or can be appropriately determined by the above-mentioned test, etc.

Specific dosage of an active ingredient to be administered in the treatment methods described herein can be determined in consideration of various conditions related to a subject requiring treatment, for example, presence or absence of conditions which inhibit regeneration, severity of symptoms, general health, age, body weight of the subject, gender of the subject, diet, time and frequency of administration, pharmaceutical agents that are used in combination, responsiveness to therapy, dosage form, and compliance to therapy.

The administration route includes various routes including both oral and parenteral routes, for example, oral, intravenous, intramuscular, subcutaneous, topical, intratumoral, rectal, intraarterial, intraportal, intraventricular, transmucosal, transdermal, intranasal, intraperitoneal, intrapulmonary, and intrauterine routes.

The frequency of administration differs depending on the properties of an agent or composition used and conditions of a subject including those described above; and the frequency may be, for example, multiple number of times per day (i.e., 2, 3, 4, or 5 times or more per day), once a day, every several days (i.e., every 2, 3, 4, 5, 6, or 7 days, etc.), every week, or every few weeks (i.e., every 2, 3, or 4 weeks).

As used herein, the term "subject" means any living individual, preferably an animal, more preferably a mammal, more preferably a human individual. In the present invention, the subject may be healthy, or may be suffering from some disease; in the case where treatment of a particular disease is contemplated, typically the subject means a subject suffering from such disease, or a subject having a risk of suffering from such disease.

In addition, the term "treatment" includes, as used herein, medically acceptable all kinds of preventive and/or therapeutic intervention for the purpose of cure, temporary remission or prevention of disease. For example, the term "treatment" includes medically acceptable intervention with variety of purposes, including delay or stop of progression of disease, regression or disappearance of lesions, prevention of onset or prevention of recurrence.

The present disclosure also relates to a cell culture substrate comprising collagen that has been treated with MMP14. The collagen treated with MMP14 is as described above with respect to various compositions.

The cell culture substrate is to be used as a scaffold for cell growth, and it may be of various forms such as membranous and gel forms. The cell culture substrate is preferably sterile, but may be provided in a non-sterile state, which will be sterilized at the time of use. The cell culture substrate may be used to coat a cell culture vessel. Coating concentration may be, without limitation, in terms of the concentration of collagen, 0.01-1000 µg/cm², 0.1-100 µg/cm², 1-50 µg/cm², or 5-25 µg/cm².

The present disclosure also relates to a kit for producing a cell culture substrate, comprising collagen that has been treated with MMP14, or comprising MMP14 and collagen. The kit may include instructions comprising information necessary to use the collagen that has been treated with MMP14 as a cell culture substrate, or information used to treat the collagen with MMP14, for example, an instruction, or an electronic recording medium such as CD, DVD, etc.

The present disclosure also relates to a cell culture vessel coated with collagen that has been treated with MMP14. As the cell culture vessel, any of known vessels can be used. The cell culture vessel may be produced by coating a cell culture vessel with collagen that has been treated with MMP14, or it may be produced by treating a collagen-pre-coated cell culture vessel with MMP14 . MMP14 used for the treatment may be attached to the cell membrane, or may be free from the cell membrane. Thus, treatment with collagen may comprise culturing cells that express MMP14, such as activated stellate cells, in a collagen-coated cell culture vessel. Cells expressing MMP14 used in the treatment with collagen may be removed after the treatment, or may be left in the cell culture vessel to which cells to be proliferated may be added. This cell culture vessel is excellent in cell proliferation, and can be used to promote cell culture. In addition, because stellate cells can induce differentiation of stem cells, a cell culture vessel which further comprises stellate cells can be used for induction of differentiation of stem cells. The coating concentration of collagen may be, without limitation, for example, 0.01-1000 µg/cm², 0.1-100 µg/cm², 1-50 µg/cm², or 5-25 µg/cm².

Furthermore, the present disclosure also relates to a kit for producing a cell culture vessel coated with collagen that has been treated with MMP14 , comprising collagen that has been treated with MMP14, or comprising MMP14 and collagen. The kit may include instructions comprising information necessary to coat a cell culture vessel with collagen that has been treated with MMP14, or information used to treat the collagen coated on the cell culture vessel with MMP14, for example, an instruction, or an electronic recording medium such as CD, DVD, etc. The kit may comprise a cell culture vessel; however, a commercially available cell culture vessel may be separately prepared and used.

The present disclosure further relates to a method for producing a cell culture vessel coated with collagen that has been treated with MMP14, comprising a step of coating a cell culture vessel coated with collagen that has been treated with MMP14, or a step of treating the collagen coated on a cell culture vessel with MMP14. MMP14 used for the treatment may be attached to the cell membrane, or may be free from the cell membrane. Thus, a step of treating the collagen coated on the cell culture vessel with MMP14 comprises culturing cells that express MMP14, such as activated stellate cells, in a collagen-coated cell culture vessel, or, contacting the collagen coated on the cell culture vessel with a decomposition product of cells expressing MMP14.

### [Examples]

The present invention is explained in further detail in the Examples below; however, they are only illustrations and do not limit the invention in any way.

### Example 1. Preparation of VA-lip siRNA

### (1) Preparation of siRNA

As the sense and antisense strands of siRNA (Hokkaido System Science Co., Ltd., Sapporo, Japan) that targets the base sequence of gp46 (GenBank Accession No. M69246, SEQ ID NO: 1), i.e., a rat homolog of human HSP47 that is a common molecular chaperone of collagen (I-IV type), the following was used:
A: GUUCCACCAUAAGAUGGUAGACAACAG (sense strand siRNA starting from the 757th base of the base sequence of gp46, SEQ ID NO: 2)
B: GUUGUCUACCAUCUUAUGGUGGAACAU (antisense strand siRNA, SEQ ID NO: 3)

As the siRNArandom (sometimes also referred to as siRNAscramble), the following was used:
C: CGAUUCGCUAGACCGGCUUCAUUGCAG (sense strand siRNA, SEQ ID NO: 4)
D: GCAAUGAAGCCGGUCUAGCGAAUCGAU (antisense strand siRNA, SEQ ID NO: 5)

In some experiments, a sense strand in which 6'-carboxyfluorescein (6-FAM) or fluorescein isothiocyanate (FITC) is bound to the 5' end was used. These sequences were confirmed to have no homology to other known rat mRNA in the BLAST search.

### (2) Preparation of VA-lip siRNA

As the cationic lipid, cationic liposomes (Lipotrust) were purchased from Hokkaido System Science Co. , Ltd. (Sapporo, Japan), which comprise O,O'-ditetradecanoyl-N-(α-trimethylammonioacetyl)diethanolami nechloride(DC-6-14), cholesterol, and dioleylphosphatidylethanolamine (DOPE) in a molar ratio of 4:3:3. Before use, the liposomes were prepared at a concentration of 1 mM (DC-6-14) by adding redistilled water (DDW) to the freeze-dried lipid mixture under stirring condition. To prepare VA-bound liposomes, 200 nmol of vitamin A (retinol, Sigma, USA) dissolved in DMSO was mixed with a liposome suspension (100 nmol as DC-6-14) in a 1.5-ml tube while stirring at 25°C. To prepare VA-bound liposomes carrying siRNAgp46 (VA-lip-siRNAgp46), a siRNAgp46 solution (580 pmol/µl in DDW) was added to a retinol-bound liposome solution while stirring at room temperature. The molar ratio of siRNA and DC-6-14 was 1:11.5, and the molar ratio of vitamin A, DC-6-14 and siRNA was 11.5:11. 5:1. To obtain desired dosage for in vitro use, VA-lip siRNA was reconstituted in phosphate-buffered saline (PBS).

### Example 2. Effect of administration of VA-lip siRNAgp46 to partial hepatectomy rat on liver regeneration

### (1) Preparation and treatment of partial hepatectomy rat

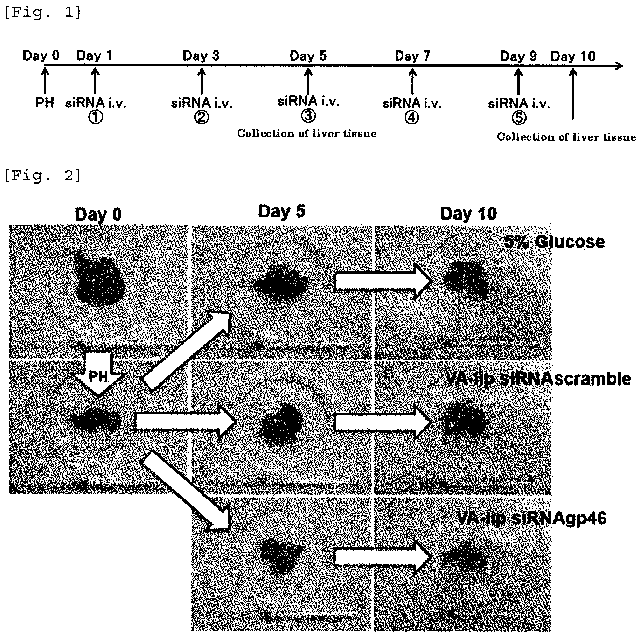

Partial hepatectomy rats were prepared by removing hepatic left and median lobes, which represents about 70% of the total liver, of male SD rats (150-200 g) (slc Japan, Shizuoka, Japan) . These partial hepatectomy rats were administered with VA-lip siRNAgp46 prepared in Example 1 (group I), VA-lip siRNAscramble (mock control, group II), or 5% glucose (siRNA-free control, group III) in a volume of 300 µl/time every other day for a total of five times (i.e., on days 1, 3, 5, 7 and 9 after partial hepatectomy) via tail vein (Fig. 1, n=6). Here, each siRNA was used at 0.75 mg/kg of rat body weight.

### (2) Evaluation of collected tissue

At 24 h after the 3^{rd} and 5th administration (i.e. , on day 5 and day 10 after partial hepatectomy), the liver of the partial hepatectomy rats was collected. After appearance of the collected liver was observed and the weight was measured, the liver was embedded using an OCT compound to prepare frozen sections. The resulting sections were fixed with 4% paraformaldehyde (PFA) and then subjected to blocking with 5% goat serum-containing PBS, washed with PBS, then allowed to react overnight using a Cy3 labeled anti-α-smooth muscle actin (a-SMA) antibody (Sigma), an anti-Ki-67 antibody (Dako), or an anti-CD133 antibody at 4°C. After washing with PBS, the sections were allowed to react using an Alexa488-labeled goat anti-mouse antibody (Invitrogen) at room temperature for 60 min. After washing with PBS, the sections were sealed with ProLong(R) Gold with DAPI (Invitrogen), and observed by fluorescence microscope. In addition, Tunel staining of the collected liver frozen sections was carried out using in situ Apoptosis Detection Kit (Takara Bio, Japan) according to the manufacturer's instructions. A FITC-TUNEL antibody and a Cy3-labeled anti-α-SMA antibody were used for co-staining of α-SMA and Tunel. The number of positive cells was calculated as the average value of five visual fields (200x magnification).

### (3) Results

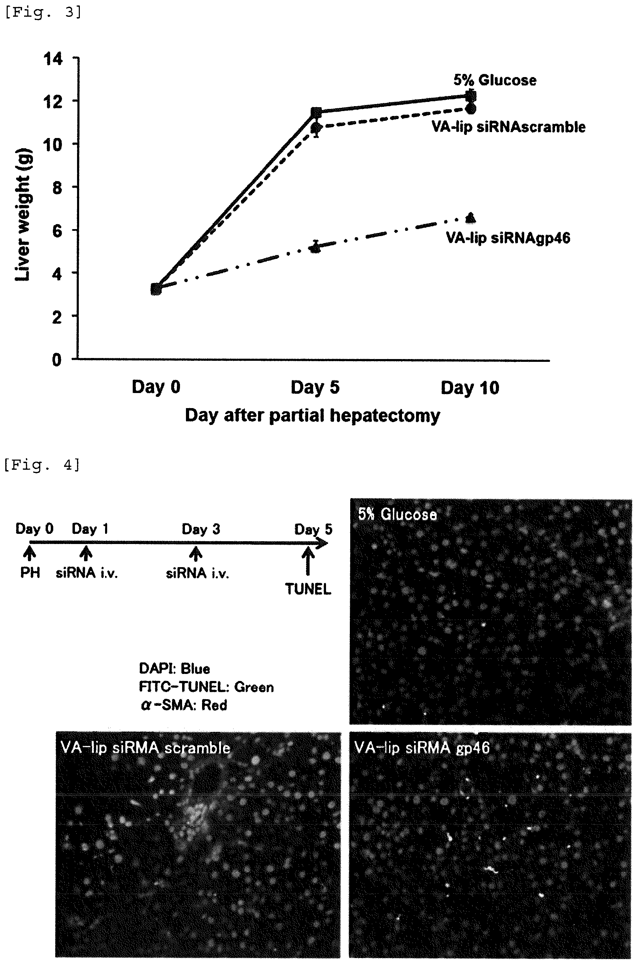

Figure 2 shows appearance of livers of each group collected on day 5 and day 10 after partial hepatectomy, in comparison to the liver before and immediately after the partial hepatectomy. In addition, Fig. 3 shows change in the weight of livers of each group collected. As can be seen from the both figures, the livers in group II and III have already recovered their previous size and weight on day 5, whereas in group I in which VA-lip siRNAgp46 was administered, the size and weight of the livers did not recover even 10 days after hepatectomy.

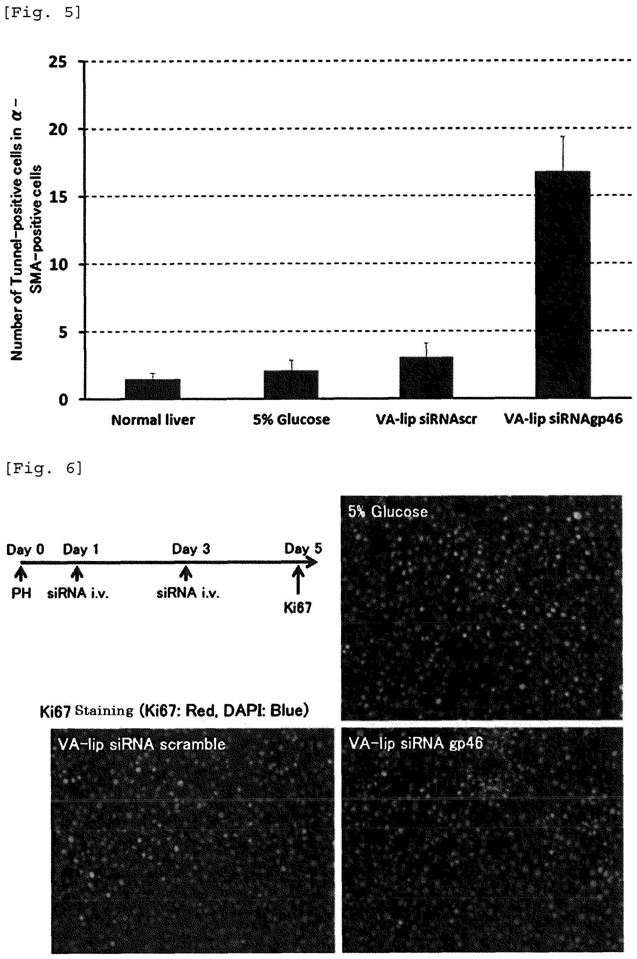

Next, liver tissues collected on day 5 after hepatectomy were co-stained with FITC-TUNEL, α-SMA and DAPI; and it was clarified that in group I, the number of TUNEL-positive cells in α-SMA positive cells was significantly larger than that in the other groups (Figs. 4 and 5). This finding indicates that large quantities of α-SMA positive cells such as activated hepatic stellate cells underwent apoptosis due to the inhibition of gp46 by VA-lip siRNAgp46.

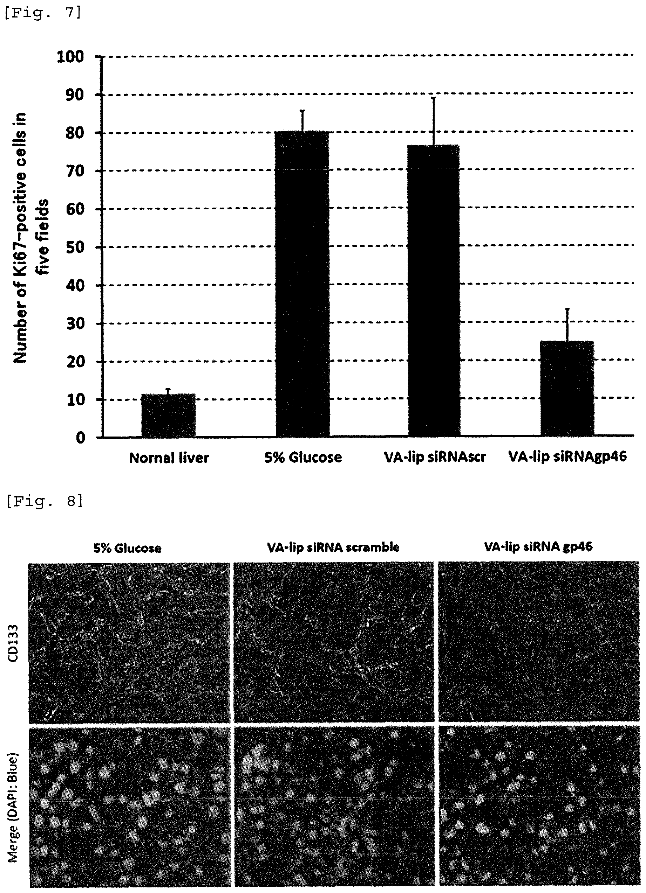

Furthermore, when the liver tissues collected on day 5 after hepatectomy were co-stained with Ki67 and DAPI, it was clarified that the number of Ki67-positive cells in group I was significantly smaller than that in the other groups subjected to the same partial hepatectomy (groups II and III) (Figs. 6 and 7) . Meanwhile, the numbers of Ki67-positive cells in groups II and III were significantly larger than that in normal liver tissue without partial hepatectomy; because Ki67 is a marker of cell proliferation, this indicates that cell proliferation was enhanced in the liver tissue after partial hepatectomy.

In addition, when the liver tissues collected on day 5 after hepatectomy were co-stained with CD133 and DAPI, it was clarified that the number of CD133-positive cells in group I was significantly smaller than that in the other groups subjected to the same partial hepatectomy (groups II and III) (Fig. 8). Since CD133 is a marker of stem cells, this result indicates that proliferation of stem cells was significantly suppressed by VA-lip siRNAgp46.

Considering the above staining results in combination, the following is indicated: due to apoptosis of α-SMA positive cells, proliferation of cells including stem cells in the liver tissue is inhibited, and, to put it the other way around, α-SMA positive cells are essential for the cell proliferation in the liver tissue toward tissue regeneration after partial hepatectomy, in particular the proliferation of stem cells.

### Example 3. Effect of administration time of VA-lip siRNAgp46 on liver regeneration in partial hepatectomy rat

### (1) Preparation and treatment of partial hepatectomy rat

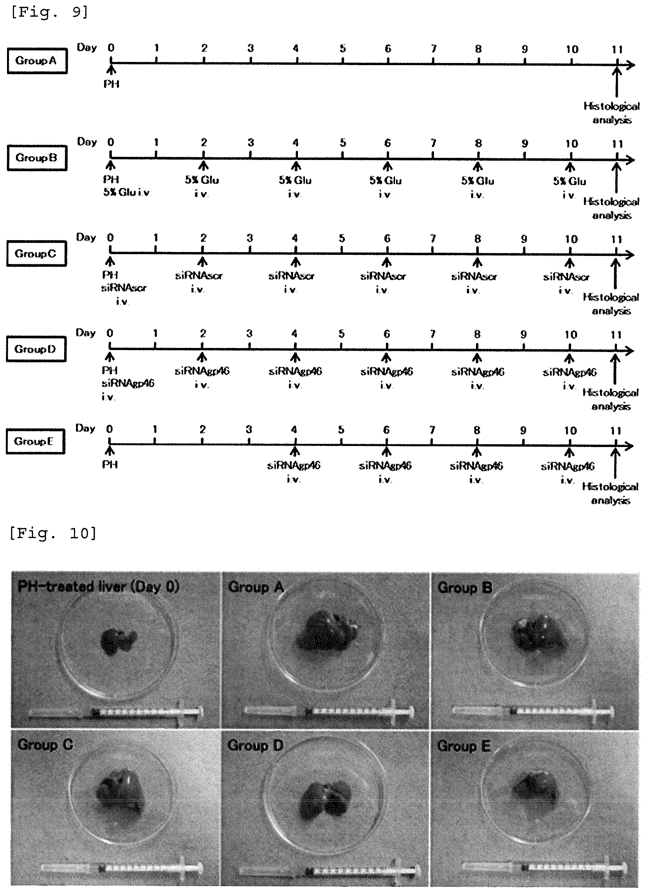

Partial hepatectomy rats were prepared similarly to Example 2. The partial hepatectomy rats were subjected to the following treatments (Fig. 9).
Group A: No treatment (non-treated control group)
Group B: 5% glucose (solvent control group)
Group C: VA-lip siRNAscramble (mock control group)
Group D: VA-lip siRNAgp46 (test group)
Group E: VA-lip siRNAgp46 (delayed administration group) (n=4 in each group)

VA-lip siRNAgp46 and VA-lip siRNAscramble prepared in Example 1 were used. In groups B-D, each of the administration substance was administered in a volume of 300 µl/time every other day for a total of six times (i.e., on days 0, 2, 4, 6, 8 and 10 after partial hepatectomy), and in group E for a total of four times (i.e., on days 4, 6, 8 and 10 after partial hepatectomy), via tail vein. In addition, each siRNA was used at 0.75 mg/kg of rat body weight.

### (2) Evaluation of collected tissue

At 24 h after completion of the 6^{th} (4^{th} for group E) administration (i.e., on day 11 after partial hepatectomy), the liver of the partial hepatectomy rats was collected. After appearance of the collected liver was observed and the weight was measured, the liver was embedded using an OCT compound to prepare frozen sections. The resulting sections were fixed with 4% paraformaldehyde and then subjected to blocking with 5% goat serum-containing PBS, washed with PBS, then allowed to react overnight using a Cy3 labeled anti-α-smooth muscle actin (a-SMA) antibody (Sigma) at 4°C. After washing with PBS, the sections were sealed with ProLong(R) Gold with DAPI (Invitrogen), and observed by fluorescence microscope.

### (3) Results

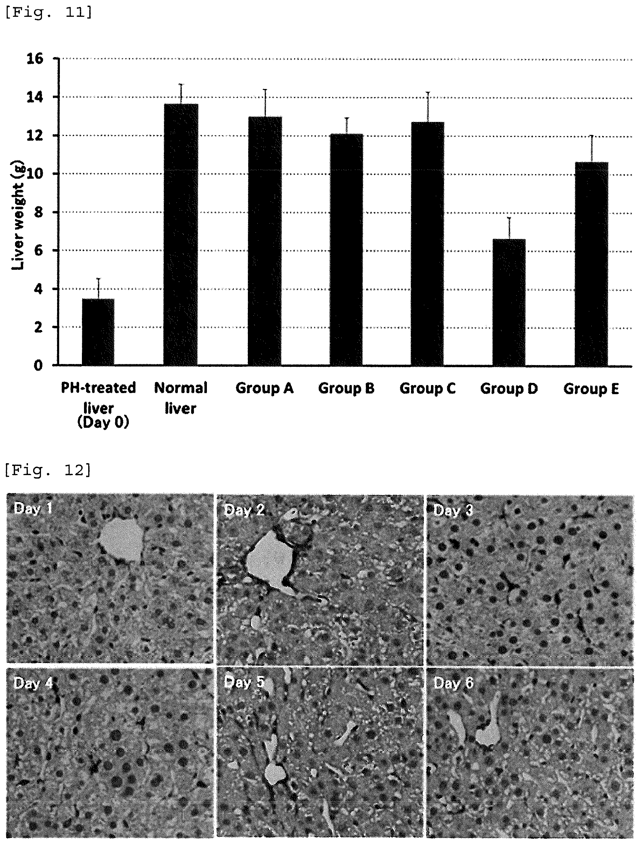

Figures 10 and 11 show appearance and weight of the livers of each group collected in the above (2), in comparison to the liver immediately after the partial hepatectomy (day 0). As can be seen from the both figures, the livers in groups A-C and E recovered their previous size and weight of before hepatectomy, whereas in group D in which VA-lip siRNAgp46 was administered starting from day 0, the size and weight of the liver did not recover even 11 days after hepatectomy. In addition, in group E in which VA-lip siRNAgp46 of the same dose was administered starting from day 4, recovery of the size and weight of the liver was observed; therefore, it was revealed that VA-lip siRNAgp46 should be administered in an early stage of tissue regeneration.

### Example 4. Time course change in the number of α-SMA-positive cells in liver tissue after partial hepatectomy

### (1) Preparation of partial hepatectomy rat and evaluation of collected tissue

Partial hepatectomy rats were prepared similarly to Example 2. From the partial hepatectomy rats, the liver was collected on day 0, 1, 2, 3, 4, 5 or 6 after partial hepatectomy. The collected liver tissue was embedded using an OCT compound to prepare frozen sections. The resulting sections were fixed with 4% paraformaldehyde and then subjected to blocking with 5% goat serum-containing PBS, washed with PBS, then allowed to react overnight using a Cy3 labeled anti-α-smooth muscle actin (a-SMA) antibody (Sigma) at 4°C. After washing with PBS, the sections were sealed with ProLong(R) Gold with DAPI (Invitrogen), and observed by fluorescence microscope.

### (2) Results

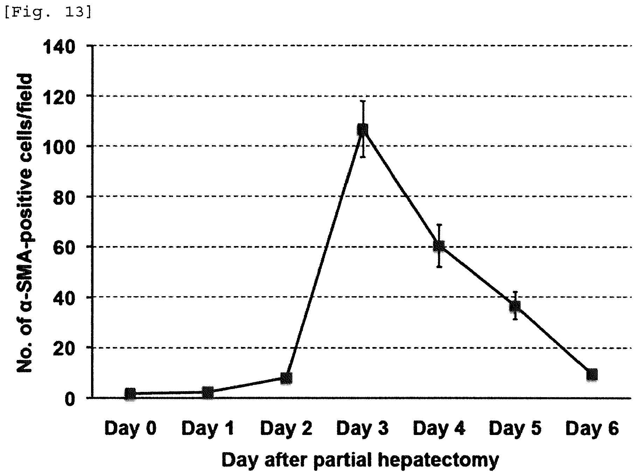

From the results shown in Figs. 12 and 13, we can see that the number of α-SMA positive cells rapidly increases on day 3 after partial hepatectomy, then gradually decreases. When considering the results of Examples 2 and 3 as well as the finding that stellate cells express α-SMA upon activation, this result suggests that stellate cells present in the tissue at an early stage of the tissue regeneration are activated, causing cell proliferation towards tissue regeneration.

### Example 5. Involvement of stellate cells in differentiation of stem cells

### (1) Preparation of cells

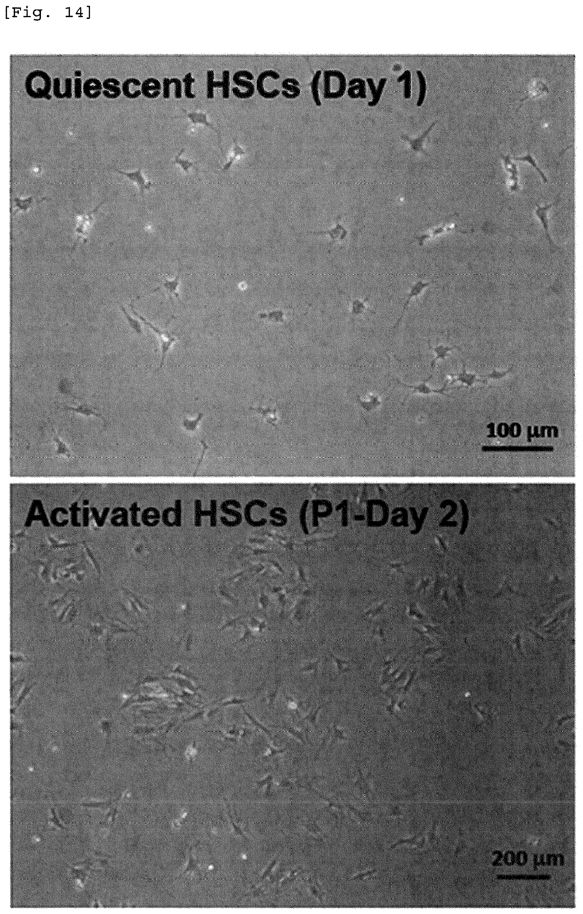

As the stellate cells, hepatic stellate cells collected from the liver of SD rats were used. That is, first, after perfusing a EGTA solution and a collagenase solution in SD rats, the liver was collected, and the collected liver was finely cut into pieces and filtered through a cell strainer (pore diameter: 100 µm). To the resulting cell suspension, a solution of HBSS + 0.25% bovine serum albumin (BSA) was added, and centrifuged at 4°C and 500 rpm for 2 min. Supernatant was collected, and centrifuged at 4°C and 1300 rpm for 5 min. After removing the supernatant, a solution of HBSS + 0.25% BSA was added, then a 28. 7% Nycodenz solution (Axis Shield, Oslo, Norway) was added and mixed to achieve the Nycodenz concentration of 13.2%. After overlaying a solution of HBSS + 0.25% BSA, is was centrifuged at 4°C and 1400 x g for 20 min. After centrifugation was completed, the intermediate layer was collected, and cultured using Dulbecco's Modified Eagle's medium (DMEM) + 10% fetal bovine serum (FBS) medium. The cells on day 1 of culturing were designated to be quiescent hepatic stellate cells (qHSCs) ; and passage culture was performed on day 5, then the cells after another 2 days of culturing were designated to be activated hepatic stellate cells (aHSCs) (Fig. 14).

As the stem cells, liver stem cells collected from the liver of 4-week-old GFP transgenic rats (Slc Japan) were used. First, after perfusing a EGTA solution and a collagenase solution in GFP transgenic rats, the liver was collected, and the collected liver was finely cut into pieces and filtered through a cell strainer (pore diameter: 100 µm). To the resulting cell suspension, Hank's balanced salt solution (HBSS) + 0.25% bovine serum albumin (BSA) solution were added, and centrifuged at 4°C and 500 rpm for 2 min. Supernatant was collected, and centrifuged at 4°C and 1300 rpm for 5 min. After removing the supernatant, Magnetic Activating Cell Sorting (MACS)® buffer (Miltenyi Biotec, Auburn, CA, USA) was added to the precipitant and mixed. The cell number was counted, then MACS® was performed using a FITC-conjugated mouse anti-CD45 antibody (BD Pharmingen), a rabbit polyclonal anti-CD133 antibody (Abcam) and a mouse monoclonal anti-EpCAM antibody (Santa Cruz); CD133-positive, EpCAM-positive and CD45-negative cells were collected and used in this experiment as the rat liver stem cells.

### (2) Contact co-culture of stem cells and stellate cells

To a 6-well plate to which type I collagen-coated coverslips (IWAKI, Tokyo, Japan) were placed, the aHSCs obtained in the above (1) were seeded at a density of 5 x 10⁴ cells/well, and cultured in an incubator at 37°C and 5% CO₂ for 48 h. Two days after aHSC seeding, the liver stem cells obtained in the above (1) were seeded on the aHSCs in the well at a density of 3 x 10⁴ cells/well, and co-cultured in the incubator at 37°C and 5% CO₂ for 9 days (as the medium, Dulbecco's Modified Eagle's Medium/Nutrient F-12 Ham (DME/F12) + 10% FBS + ITS (10 mg/l insulin, 5.5 mg/l transferrin, 0.67 µg/l selenium) + 0.1 µM dexamethasone + 10 mM nicotinamide + 50 µg/ml β-mercaptoethanol + 2 mM L-glutamine + 5 mM Hepes was used). Depending on the conditions, 20 ng/ml of EGF and/or 50 ng/ml of HGF were added at the start of co-culture. As a control, liver stem cells alone were similarly cultured without aHSC.

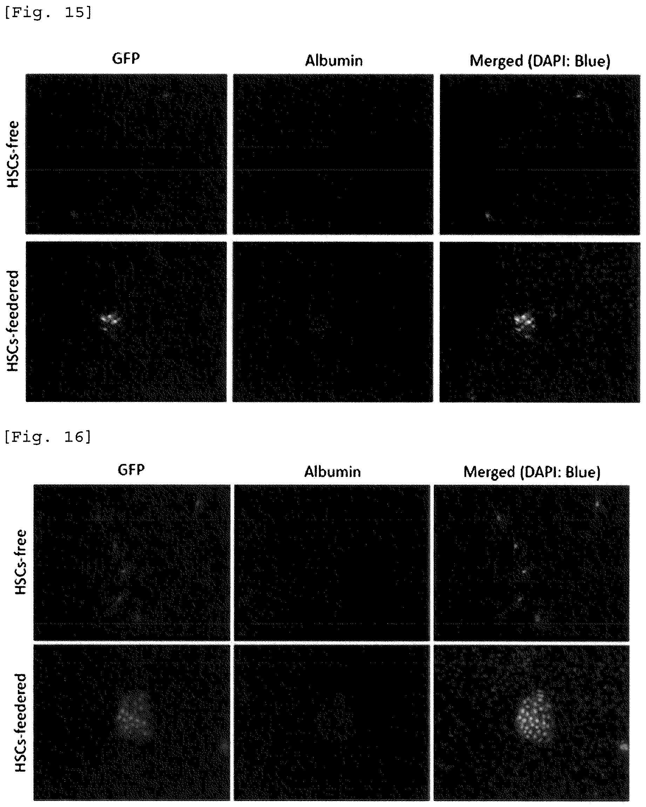

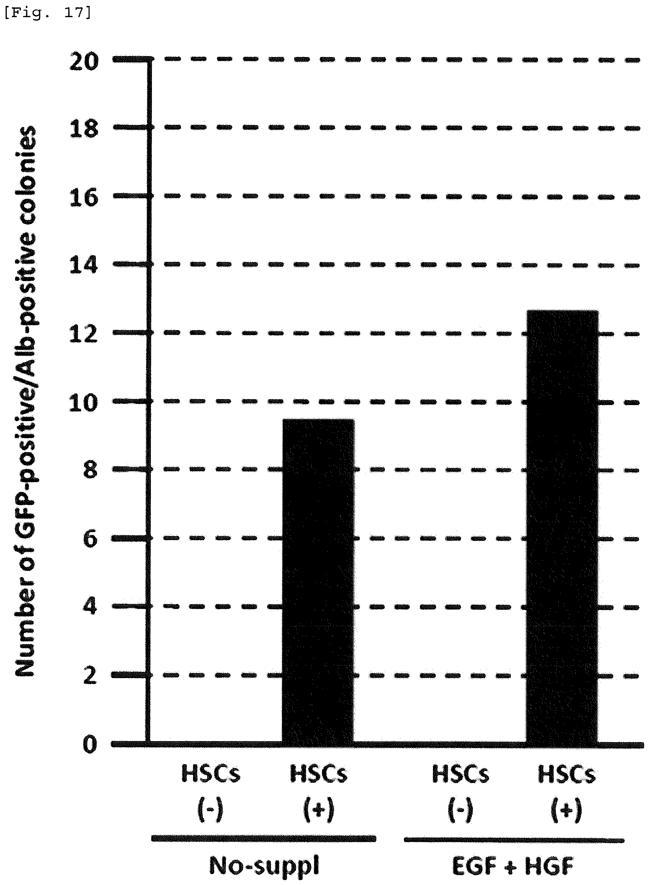

On day 9 of co-culturing, immunostaining with an antibody (rabbit polyclonal, MP Biomedicals) against albumin, i.e., a liver cell marker, was performed, and GFP-positive/albumin-positive colonies were photographed using an inverted microscope (Nikon) at 100x magnification, then, from the images obtained, the number of GFP-positive/albumin-positive colonies was counted, and the area was calculated using NIS-Elements software (Nikon) (Figs. 15-18).

### (3) Non-contact co-culture of stem cells and stellate cells

The aHSCs obtained in the above (1) were seeded at a density of 5 x 10⁴ cells/well on a cell culture insert (pore size: 0.4 µm, BD Falcon, Franklin Lakes, NJ, USA), and cultured in an incubator at 37°C and 5% CO₂ for 48 h using DMEM + 10% FBS. Two days after aHSC seeding, the liver stem cells obtained in the above (1) were seeded on a 24-well plate to which type I collagen-coated coverslips (IWAKI, Tokyo, Japan) were placed, at a density of 1 x 10⁴ cells/well. Then, the above cell culture insert containing aHSCs was inserted in the wells of the 24-well plate, and subjected to co-culturing in an incubator at 37°C and 5% CO₂ for 10 days (as the medium, Dulbecco's Modified Eagle's Medium/Nutrient F-12 Ham (DME/F12) + 10% FBS + ITS (10 mg/l insulin, 5.5 mg/l transferrin, 0.67 µg/l selenium) + 0.1 µM dexamethasone + 10 mM nicotinamide + 50 µg/ml β-mercaptoethanol + 2 mM L-glutamine + 5 mM Hepes was used). As a control, liver stem cells alone were similarly cultured without aHSC.

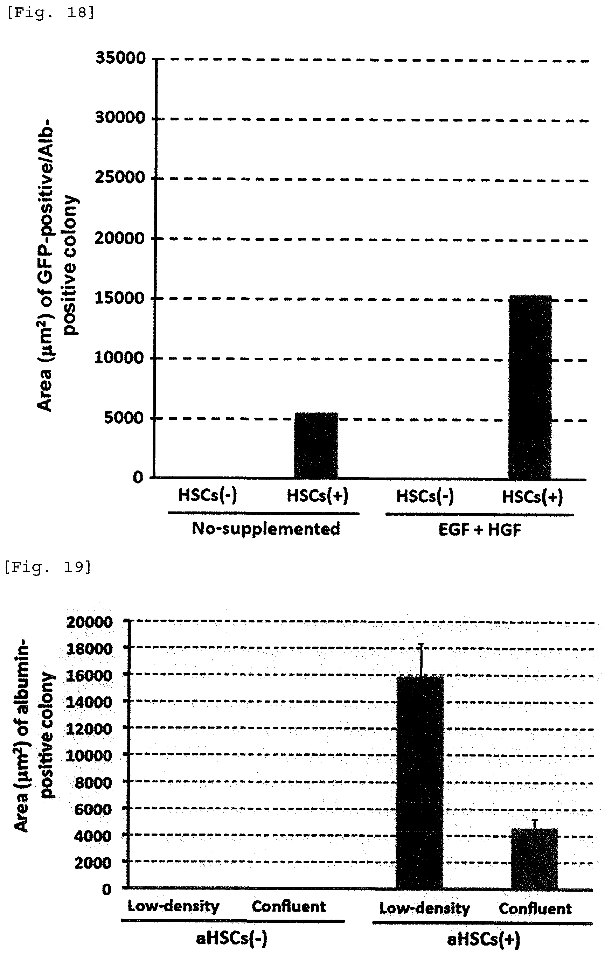

On day 10 of co-culturing, immunostaining with an anti-albumin antibody (rabbit polyclonal, MP Biomedicals) was performed and albumin positive colonies were photographed using an inverted microscope (Nikon) at 100x magnification, and from the images obtained, the area of the albumin positive colonies was calculated using NIS-Elements software (Nikon) (Fig. 19).

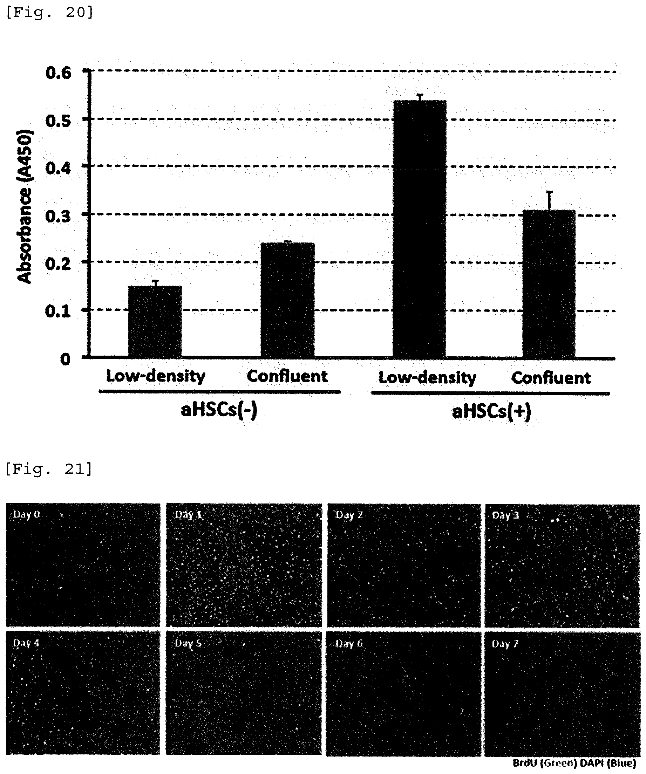

In another experiment, on day 10 of co-culturing, cell proliferation was measured using Premix WST-1 Cell Proliferation Assay System (Takara, Tokyo, Japan) by a microplate reader (Bio-Rad Laboratories, Hercules, CA, USA) (Fig. 20).

### (4) Results

Results of the contact co-culture experiments shown in Figs. 15-18 revealed that differentiation and proliferation of liver stem cells into liver cells occur due to co-culturing of liver stem cells with activated hepatic stellate cells. In addition, it was clarified that the differentiation and proliferation of liver stem cells into liver cells were significantly promoted by the addition of EGF and HGF.

Results of the non-contact co-culture experiments shown in Figs. 19 and 20 revealed that activated hepatic stellate cells are capable of inducing differentiation and proliferation of liver stem cells into liver cells, even when they are not in contact with the liver stem cells.

These results indicate that the humoral factors secreted from activated hepatic stellate cells induce differentiation and proliferation of liver stem cells into liver cells.

### Example 6. Time course change in DNA synthesis in liver tissue after partial hepatectomy

### (1) Time course change in BrdU-positive cells

Partial hepatectomy rats were prepared similarly to Example 2. To investigate time course change in DNA synthesis in the liver after partial hepatectomy, the rats were intraperitoneally injected with BrdU at a concentration of 100 µg/g of body weight. The liver tissue was collected at 3 h after the BrdU administration. The collected liver tissue was fixed in 10% formalin, and embedded in paraffin. The paraffin-embedded liver tissue was sectioned into a thickness of 5 µm, activated using a 10 mM citrate buffer at 120°C for 20 min, then blocked with 5% goat serum. After blocking, it was allowed to react with a mouse monoclonal anti-BrdU antibody (MBL) at 37°C for 60 min. The sections were washed with PBS, then allowed to react with Alexa488-labeled goat anti-mouse IgG (Invitrogen) at 37°C for 60 min. After washing with PBS, the sections were sealed with a mounting agent containing DAPI, and observed for the incorporation of BrdU in the liver tissue. Results are shown in Fig. 21.

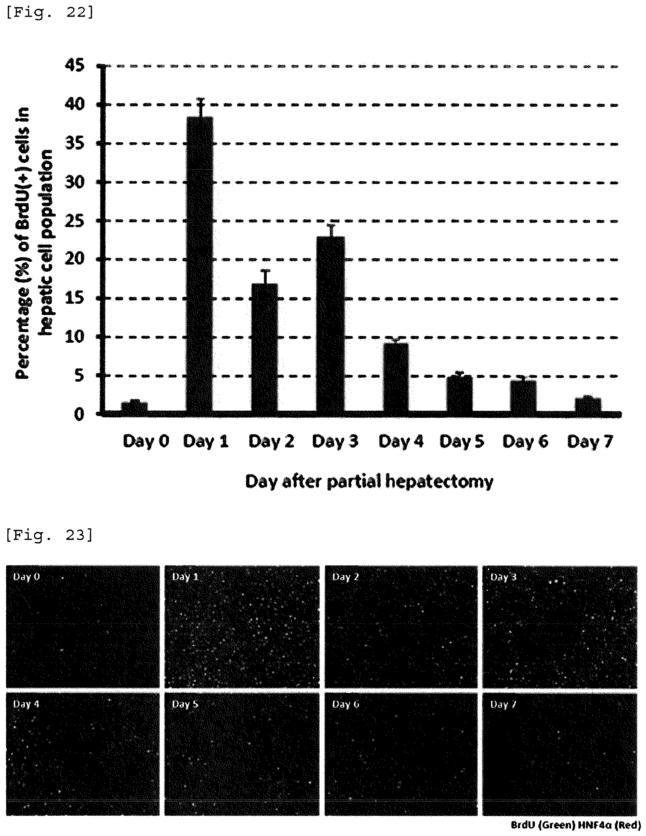

To determine the percentage of cells in which DNA synthesis occurs in the liver tissue of rats after partial hepatectomy, the rats subjected to partial hepatectomy were intraperitoneally injected with BrdU at a concentration of 100 µg/g of body weight. At 3 h after the BrdU administration, the rats were perfused with a 0.03% collagenase solution from the portal vein, then the liver tissue was collected and finely cut into pieces to obtain a cell suspension. The cell concentration of this cell suspension was adjusted with Hank's solution containing 0.25% BSA (Invitrogen) at 1 x 10⁷ cells/100 µl, and an APC-labeled mouse monoclonal anti-BrdU antibody (BioLegends) was added and allowed to react at 37°C for 60 min. After completion of the reaction, the number of BrdU-positive cells was measured using flow cytometry. Figure 22 shows the results. In the figure, the percentage of BrdU-positive cells in the liver of rats after partial hepatectomy was expressed by a number of BrdU-positive cells divided by the total number of cells analyzed by the flow cytometry.

From the results shown in Figs. 21 and 22, we can see that after partial hepatectomy, DNA synthesis in the liver tissue increases in a bimodal manner. In other words, DNA synthesis shows a first peak on day 1 after hepatectomy, then it decreases slightly on day 2, increases again on day 3, and gradually decreases on day 4 and after.

### (2) Time course change in the number of BrdU-positive liver cells

Partial hepatectomy rats were prepared similarly to Example 2. To investigate time course change in DNA synthesis in liver cells of the liver after partial hepatectomy, the rats were intraperitoneally injected with BrdU at a concentration of 100 µg/g of body weight. The liver tissue was collected at 3 h after the BrdU administration. The collected liver tissue was fixed in 10% formalin, and embedded in paraffin. The paraffin-embedded liver tissue was sectioned into a thickness of 5 µm, activated using a 10 mM citrate buffer at 120°C for 20 min, and blocked with 5% goat serum. After blocking, it was allowed to react with an APC-labeled mouse monoclonal anti-BrdU antibody (BioLegends) and a goat anti-HNF4α antibody (Santa Cruz) at 37°C for 60 min. After washing with PBS, the sections were allowed to react with Alexa488-labeled sheep anti-goat IgG (Invitrogen) at 37°C for 60 min. After washing with PBS, the sections were sealed with a mounting agent containing DAPI, and observed for the incorporation of BrdU by liver cells in the liver tissue. Results are shown in Fig. 23.

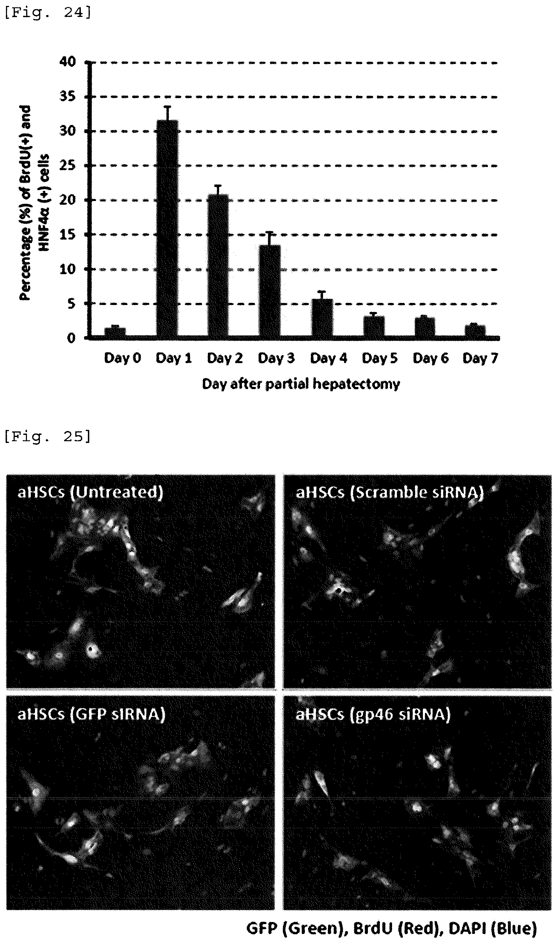

To determine the percentage of liver cells in which DNA synthesis occurs in the liver tissue of rats after partial hepatectomy, the rats subjected to partial hepatectomy were intraperitoneally injected with BrdU at a concentration of 100 µg/g of body weight. At 3 h after the BrdU administration, the rats were perfused with a 0.03% collagenase solution from the portal vein, then the liver tissue was collected and finely cut into pieces to obtain a cell suspension. The cell concentration of this cell suspension was adjusted with Hank's solution containing 0.25% BSA (Invitrogen) at 1 x 10⁷ cells/100 µl, and an APC-labeled mouse monoclonal anti-BrdU antibody (BioLegends) and a goat anti-HNF4α antibody (Santa Cruz) were added and allowed to react at 37°C for 60 min. After washing with PBS, it was allowed to react with Alexa488-labeled sheep anti-goat IgG (Invitrogen) at 37°C for 60 min. After completion of the reaction, flow cytometry was used to measure the number of BrdU-positive HNF4α-positive cells. Results are shown in Fig. 24. In the figure, the percentage of BrdU-positive liver cells in the liver of rats after partial hepatectomy was expressed by a number of BrdU-positive liver cells divided by the total number of cells analyzed by the flow cytometry.

From the results shown in Figs. 23 and 24, we can see that the proliferation of liver cells peaks on 1 day after the partial hepatectomy, then decreases gradually. Proliferation of all the cells present in the liver including hepatic cells has two peaks on day 1 and day 3 after partial hepatectomy, as shown in Figs. 21 and 22, and it is considered that the first peak on day 1 is due mainly to the proliferation of liver cells, whereas the second peak on day 3 is due to the proliferation of cells other than liver cells, for example, stem cells, etc.

### Example 7. Involvement of stellate cells in proliferation of liver cells

### (1) Co-culture of liver cells and stellate cells

To evaluate effects of stellate cells on proliferation of liver cells, co-culturing of liver cells and stellate cells was performed. The aHSCs obtained in Example 5 were seeded on 60-mm dishes at a concentration of 1 x 10⁵ cells, and cultured using 10% FBS-added DMEM at 37°C and 5% CO₂ for 24 h. At 24 h of culturing, the medium was removed and Opti-MEM I reducing medium (Invitrogen) was added, and the cells were pre-cultured until ready for transfection of siRNA. siRNA was mixed with RNAiMAX (Invitrogen) to achieve the final siRNA concentration of 10 nM, and they were allowed to stand at room temperature for 15 min to obtain siRNA complexes. The following types of SiRNA were used. GFP siRNA (Ambion, Silencer GFP siRNA, Cat. No.AM4626) Scramble siRNA: those described in Example 1 gp46 siRNA: those described in Example 1

The siRNA complexes were added to the pre-cultured aHSCs, and cultured at 37°C and 5% CO₂ for 5 h. At 5 h of culturing, the medium was replaced with 10% FBS-added DMEM, and cultured at 37°C and 5% CO₂ for 48 h. At 48 h of culturing, the medium was removed and Opti-MEM I reducing medium was added, and the cells were pre-cultured until ready for transfection of siRNA. siRNA (GFP siRNA, Scramble siRNA, gp46 siRNA) was mixed with RNAiMAX to achieve the final siRNA concentration of 10 nM, and they were allowed to stand at room temperature for 15 min. The siRNA complexes were added to the pre-cultured aHSCs, and cultured at 37°C and 5% CO₂ for 5 h. The medium was removed at 5 h of culturing, and the cells were washed with PBS, and HSCs were recovered using a trypsin-EDTA solution.

The recovered HSCs were added at a concentration of 5 x 10⁴ cells/dish to the liver cells which were collected in advance from GFP transgenic rats and seeded at a concentration of 5 x 10⁴ cells/dish on a 35-mm dish coated with collagen I; and cultured at 37°C and 5% CO₂ for 48 h. At 48 h of culturing, the medium was replaced with DME/F12 medium containing 10 mM BrdU and 10% FBS, and cultured at 37°C and 5% CO₂ for another 24 h. At 24 h of culturing, the medium was removed, the cells were washed with PBS, to which 4% PFA was added and fixed at room temperature for 30 min. After fixing, the cells were washed with PBS, and permeabilized by the addition of PBS containing 0.2 N HCl and 0.1% Triton X-100.

### (2) Observation by fluorescence microscope

The cells permeabilized in the above (1) were washed with PBS, and subjected to blocking with 5% goat serum, and a primary antibody reaction was performed at 37°C for 60 min by adding a mouse monoclonal anti-BrdU antibody (MBL) and a rabbit polyclonal anti-GFP antibody (Invitrogen). After the primary antibody reaction, the cells were washed with PBS, and a secondary antibody reaction was performed at 37°C for 60 min by adding Alexa488-labeled goat anti-rabbit IgG (Invitrogen) and Alexa555-labeled goat anti-mouse IgG (Invitrogen). After the secondary antibody reaction, the cells were washed with PBS and sealed with a mounting agent containing DAPI. Fluorescence microscope images are shown in Fig. 25.

### (3) FACS analysis

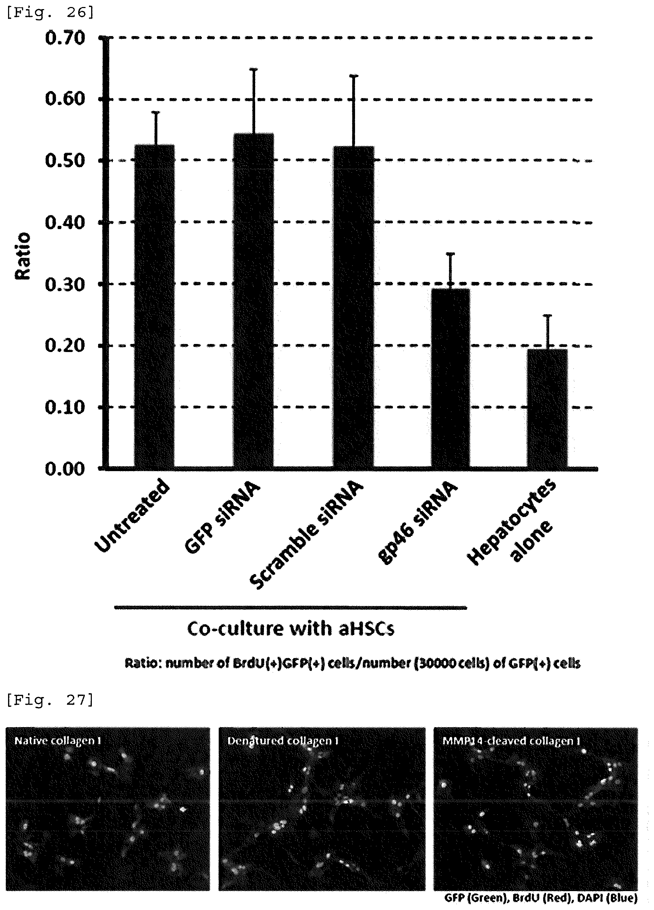

To determine the ratio of BrdU-positive GFP-positive liver cells, the cells permeabilized in the above (1) were washed with PBS, and an antibody reaction was performed at 37°C for 60 min by adding an APC-labeled mouse monoclonal anti-BrdU antibody and a FITC-labeled rabbit polyclonal anti-GFP antibody. After completion of the antibody reaction, the cells were washed with PBS, and the ratio of BrdU-positive GFP-positive liver cells was determined by FACS analysis. Results are shown in Fig. 26. The ratio of BrdU-positive cells was expressed by the number of BrdU-positive cells divided by the total number of cells analyzed by the flow cytometry.

From the results shown in Figs. 25 and 26, we can see that, when liver cells are co-cultured with aHSCs, proliferation of the liver cells is enhanced compared to the case in which liver cells are cultured alone, and this effect is suppressed when gp46 siRNA is reacted to the aHSCs. These results indicate that aHSCs are involved in the proliferation of liver cells.

### Example 8. Effects of collagen which has been subjected to various treatments on proliferation of liver cells

### (1) Culturing liver cells on collagen which has been subjected to various treatments

Rat tail-derived collagen I (Sigma) was coated on a 60-mm dish at a concentration of 10 µg/cm². Denatured collagen I was prepared by treating the rat tail-derived collagen I at 60°C for 30 min, and was coated on a 60-mm dish at a concentration of 10 µg/cm². MMP14-treated collagen I was prepared by reacting the rat tail-derived collagen I using a reaction solution (0.1 µg/ml trypsin 3 (Recombinant Human Active Trypsin3, R & D systems, Cat. No. 3714-SE), 50 mM Tris, 0.15 M NaCl, 10 mM CaCl₂, 5 µM ZnCl₂, 0.05% (v/v) Brij35, pH 7.5) containing active-type MMP14 (R & D System), at room temperature for 20 h. After completion of the reaction, MMP14-treated collagen I was coated on a 60-mm dish at a concentration of 10 µg/cm². Liver cells were collected from the liver of a rat, seeded at a concentration of 2 x 10⁵ cells/dish, and cultured at 37°C and 5% CO₂ for 48 h. At 48 h of culturing, the medium was replaced with DME/F12 medium containing 10 µM BrdU and 10% FBS, and cultured at 37°C and 5% CO₂ for another 24 h. At 24 h of culturing, the medium was removed, the cells were washed with PBS, and fixed by the addition of 4% PFA at room temperature for 30 min. After fixing, the cells were washed with PBS, and permeabilized by the addition of PBS containing 0.2 N HCl and 0.1% Triton X-100.

### (2) Observation by fluorescence microscope

The cells permeabilized in the above (1) were washed with PBS, and subjected to blocking with 5% goat serum, and a primary antibody reaction was performed at 37°C for 60 min by adding a mouse monoclonal anti-BrdU antibody (MBL) and a rabbit polyclonal anti-GFP antibody (Invitrogen). After the primary antibody reaction, the cells were washed with PBS, and a secondary antibody reaction was performed at 37°C for 60 min by adding Alexa488-labeled goat anti-rabbit IgG (Invitrogen) and Alexa555-labeled goat anti-mouse IgG (Invitrogen). After the secondary antibody reaction, the cells were washed with PBS and sealed with a mounting agent containing DAPI. Fluorescence microscope images are shown in Fig. 27.

### (3) FACS analysis

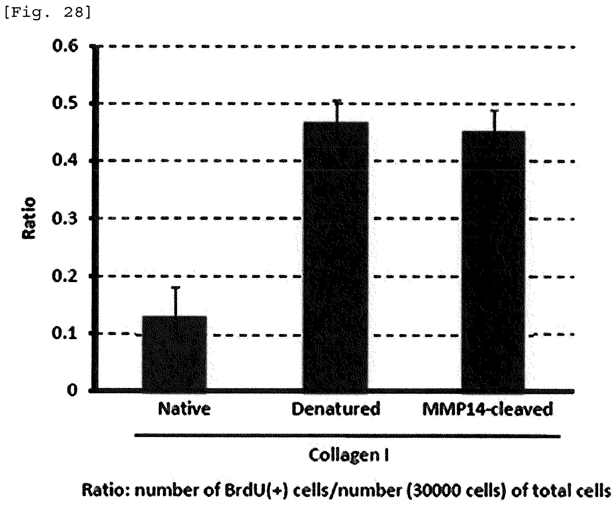

To determine the ratio of BrdU-positive liver cells, the cells permeabilized in the above (1) were washed with PBS, and an antibody reaction was performed at 4°C for 30 min by adding an APC-labeled mouse monoclonal anti-BrdU antibody. After completion of the antibody reaction, the cells were washed with PBS, and the ratio of BrdU-positive liver cells was determined by FACS analysis. Results are shown in Fig. 28. The ratio of BrdU-positive cells was expressed by the number of BrdU-positive cells divided by the total number of cells (3 x 10⁴ cells) analyzed by the flow cytometry.

From the results shown in Figs. 27 and 28, we can see that, proliferation of the liver cells was promoted in the dish coated with denatured collagen I and MMP14-treated collagen I, compared to the dish coated with untreated collagen. Since MMP14 having a collagenase activity is expressed in HSCs, the above results suggest that the action of MMP14 expressed by HSCs on the collagen is involved as a cause of promoting proliferation of liver cells by HSCs.

### Example 9. Effects of MMP14 expression in aHSCs on proliferation of liver cells

### (1) Co-culture of liver cells and stellate cells

To evaluate effects of stellate cells on the proliferation of liver cells, co-culturing of liver cells and stellate cells was performed. The aHSCs obtained in Example 5 were seeded on 60-mm dishes at a concentration of 1 x 10⁵ cells, and cultured using 10% FBS-added DMEM at 37°C and 5% CO₂ for 24 h. At 24 h of culturing, the medium was removed and Opti-MEM I reducing medium (Invitrogen) was added, and the cells were pre-cultured until ready for transfection of siRNA. siRNA was mixed with RNAiMAX (Invitrogen) to achieve the final siRNA concentration of 10 nM, and the cells were allowed to stand at room temperature for 15 min to obtain siRNA complexes . The following types of siRNA were used.
GFP siRNA (Ambion, Silencer GFP siRNA, Cat. No.AM4626)
MMP14 siRNA:
Sense strand: 5'-GCUCAUUCAUGGGUAGCGATT-3'(SEQ ID NO: 6)
Antisense strand: 5'-UCGCUACCCAUGAAUGAGCCT-3'(SEQ ID NO: 7) HGF siRNA:
Sense strand: 5'-AUAUCUUUCCGGCAAGAAUUUGUGC-3'(SEQ ID NO: 8)
Antisense strand: 5'-GCACAAAUUCUUGCCGGAAAGAUAU-3' (SEQ ID NO: 9)

The siRNA complexes were added to the pre-cultured aHSCs and cultured at 37°C and 5% CO₂ for 5 h. At 5 h of culturing, the medium was replaced with 10% FBS-added DMEM, and cultured at 37°C and 5% CO₂ for 48 h. At 48 h of culturing, the medium was removed and Opti-MEM I reducing medium was added, and the cells were pre-cultured until ready for transfection of siRNA. siRNA (GFP siRNA, MMP14 siRNA, HGF siRNA) was mixed with RNAiMAX to achieve the final siRNA concentration of 10 nM, and the cells were allowed to stand at room temperature for 15 min. The siRNA complexes were added to the pre-cultured aHSCs, and cultured at 37°C and 5% CO₂ for 5 h. The medium was removed at 5 h of culturing, and the cells were washed with PBS, and HSCs were recovered using a trypsin-EDTA solution.

The recovered HSCs were added at a concentration of 5 x 10⁴ cells/dish to the liver cells which were collected in advance from GFP transgenic rats and seeded at a concentration of 5 x 10⁴ cells/dish on 35-mm dishes coated with collagen I, and cultured at 37°C and 5% CO₂ for 48 h. At 48 h of culturing, the medium was replaced with DME/F12 medium containing 10 mM BrdU and 10% FBS, and cultured at 37°C and 5% CO₂ for another 24 h. At 24 h of culturing, the medium was removed, the cells were washed with PBS, to which 4% PFA was added and fixed at room temperature for 30 min. After fixing, the cells were washed with PBS, and permeabilized by the addition of PBS containing 0.2 N HCl and 0.1% Triton X-100.

### (2) Observation by fluorescence microscope

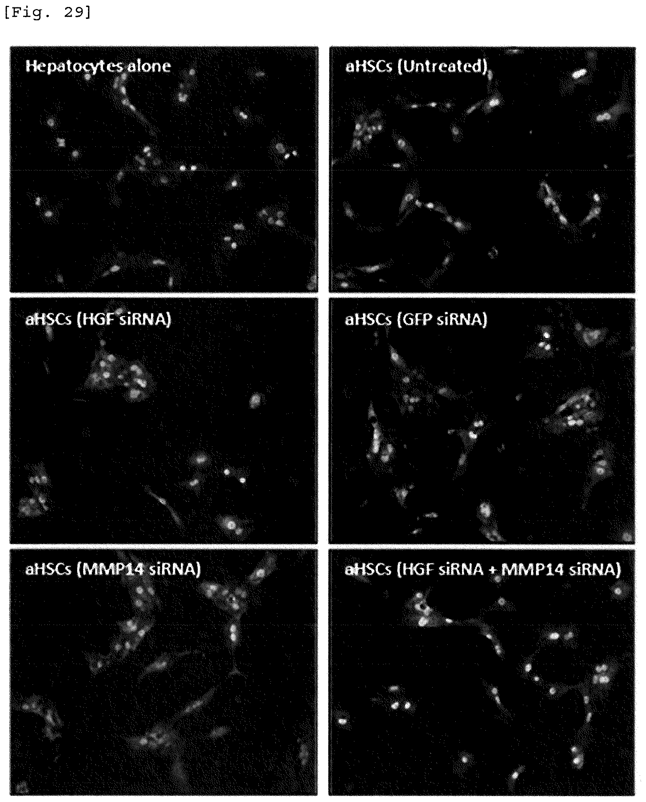

The cells permeabilized in the above (1) were washed with PBS, and subjected to blocking with 5% goat serum, and a primary antibody reaction was performed at 37°C for 60 min by adding a mouse monoclonal anti-BrdU antibody (MBL) and a rabbit polyclonal anti-GFP antibody (Invitrogen). After the primary antibody reaction, the cells were washed with PBS, and a secondary antibody reaction was performed at 37°C for 60 min by adding Alexa488-labeled goat anti-rabbit IgG (Invitrogen) and Alexa555-labeled goat anti-mouse IgG (Invitrogen). After the secondary antibody reaction, the cells were washed with PBS and sealed with a mounting agent containing DAPI. Fluorescence microscope images are shown in Fig. 29.

### (3) FACS analysis

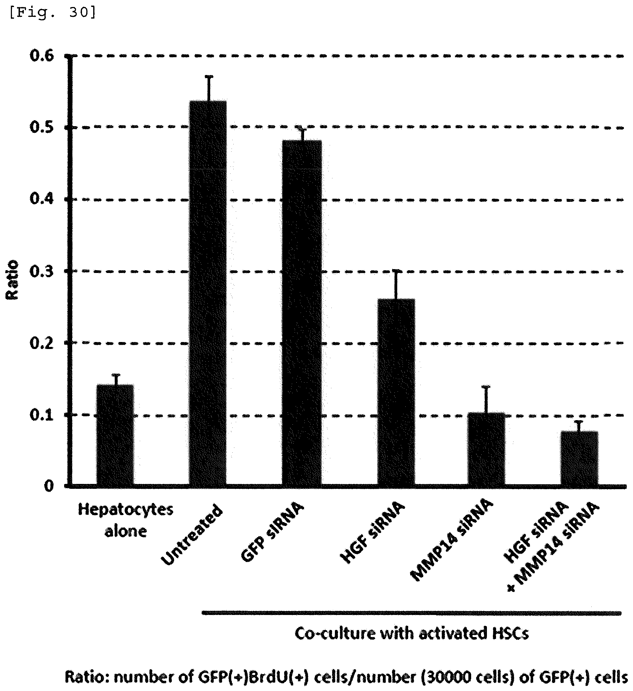

To determine the ratio of BrdU-positive GFP-positive liver cells, the cells permeabilized in the above (1) were washed with PBS, and an antibody reaction was performed at 4°C for 30 min by adding an APC-labeled mouse monoclonal anti-BrdU antibody and a FITC-labeled rabbit polyclonal anti-GFP antibody,. After completion of the antibody reaction, the cells were washed with PBS, and the ratio of BrdU-positive GFP-positive liver cells was determined by FACS analysis. Results are shown in Fig. 30. The ratio of BrdU-positive cells was expressed by the number of BrdU-positive cells divided by the total number of cells analyzed by the flow cytometry.

From the results shown in Figs. 29 and 30, we can see that MMP14 expressed by aHSCs is involved in promoting proliferation of hepatocytes by aHSCs, and that a degree of contribution of MMP14 is greater than that of HGF, which is known as a key factor in promoting proliferation of hepatocytes.

### Example 10. Effects of RGD sequence on MMP14-treated collagen on proliferation of liver cells

Rat liver cells collected from the livers of GFP transgenic rats were seeded on DME/F12 medium at a concentration of 2 x 10⁵ cells/ml, and different concentrations of peptides (control peptide (H-Gly-Arg-Gly-Glu-Glu-Ser-OH, Peptides International, Cat. No. PFA-3907-PI): 500 µg/ml, GRGDS peptide (Peptide Institute, Cat. No. 4189-v) : 100 µg/ml, 200 µg/ml, 500 µg/ml) were added, and allowed to react at 37°C for 30 min. The rat liver cells after completion of the reaction were seeded at a concentration of 5 x 10⁴ cells/dish on 35-mm dishes coated with MMP14-treated collagen I, cultured on DME/F12 medium containing 10 mM BrdU and 10% fetal bovine serum at 37°C and 5% CO₂ for 72 h. After completion of the culture, the medium was removed, the cells were washed with PBS, and fixed by the addition of 4% PFA at room temperature for 30 min. After fixing, the cells were washed with PBS and permeabilized by the addition of PBS containing 0.2 N HC1 and 0.1% Triton X-100.

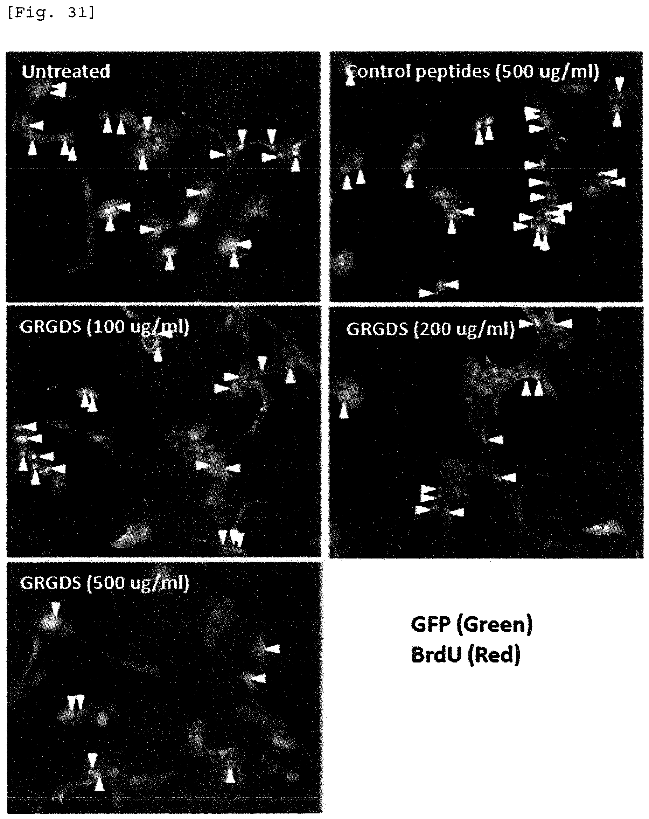

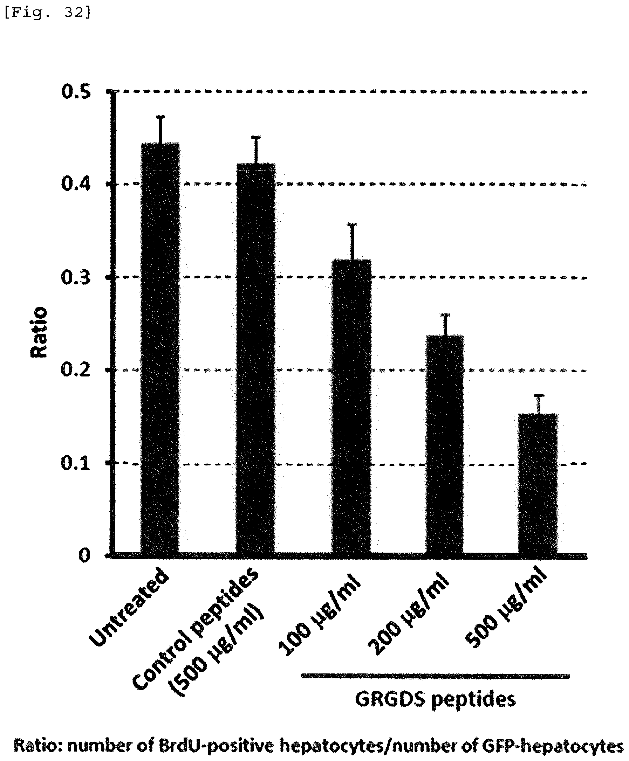

After washing with PBS and subjected to blocking with 5% goat serum, a primary antibody reaction was performed at 37°C for 60 min by adding a mouse monoclonal anti-BrdU antibody (MBL) and a rabbit polyclonal anti-GFP antibody (Invitrogen). After the primary antibody reaction, the cells were washed with PBS, and a secondary antibody reaction was performed at 37°C for 60 min by adding Alexa488-labeled goat anti-rabbit IgG (Invitrogen) and Alexa555-labeled goat anti-mouse IgG (Invitrogen). After the secondary antibody reaction, the cells were washed with PBS and sealed with a mounting agent containing DAPI. Fluorescence microscope images are shown in Fig. 31. The ratio of cell proliferation was expressed by the number of BrdU-positive cells divided by the total number of cells counted (Fig. 32).

From the results shown in Figs. 31 and 32, we can see that RGD sequence is involved in promoting proliferation of hepatocytes by aHSCs. This suggests that the RGD sequence of collagen I exposed by the action of MMP14 expressed by aHSCs is involved in promoting proliferation of hepatocytes.

## Claims

1. A composition for use in promoting tissue regeneration of liver in a subject, the composition comprising a collagen that has been treated with MMP14, wherein the collagen is in a condition where RGD sequence is ready to interact with cells.

2. The composition for use according to Claim 1, wherein the tissue regeneration occurs at damaged tissue or transplanted tissue, optionally the composition is administered within four days, preferably within one day from the day of receiving damage or transplantation.

3. The composition for use according to Claim 2, wherein the damage is selected from tissue destruction, inflammation, necrosis, fibrosis, surgical invasion and organ failure.

4. The composition for use according to any one of Claims 1 to 3, wherein the tissue regeneration involves differentiation and/or proliferation of tissue stem cells.

5. The composition for use according to any one of Claims 1 to 4, wherein the tissue regeneration involves proliferation of tissue parenchymal cells.

6. The composition for use according to any one of Claims 1 to 5, wherein the subject has a condition in which tissue regeneration is suppressed, preferably the condition of suppressed tissue regeneration is selected from the group consisting of inflammation, necrosis, fibrosis, organ failure, decreased platelet count, genetic abnormalities, and reduction of noradrenaline.

7. A composition for use in differentiation and/or proliferation of liver stem cells in a subject, the composition comprising a collagen that has been treated with MMP14, wherein the collagen is in a condition where RGD sequence is ready to interact with cells.

8. A method for differentiation and/or proliferation of liver stem cells in vitro, comprising a step of contacting a collagen that has been treated with MMP14 with the liver stem cells, wherein the collagen is in a condition where RGD sequence is ready to interact with cells.

9. A composition for use in promoting liver cell proliferation in a subject, the composition comprising a collagen that has been treated with MMP14, wherein the collagen is in a condition where RGD sequence is ready to interact with cells.

10. A method for promoting liver cell proliferation in vitro, comprising a step of contacting a collagen that has been treated with MMP14 with the liver cell, wherein the collagen is in a condition where RGD sequence is ready to interact with cells.

## Patentansprüche

1. Zusammensetzung zur Verwendung beim Fördern der Geweberegeneration der Leber in einem Subjekt, wobei die Zusammensetzung ein Kollagen umfasst, das mit MMP14 behandelt wurde, wobei das Kollagen in einem Zustand ist, in dem RGD-Sequenz bereit ist, mit Zellen zu interagieren.

2. Zusammensetzung zur Verwendung gemäß Anspruch 1, wobei die Geweberegeneration an beschädigtem Gewebe oder transplantiertem Gewebe erfolgt, wobei optional die Zusammensetzung innerhalb von vier Tagen, bevorzugt innerhalb eines Tages ab dem Tag, an dem es Beschädigung oder Transplantation erhielt, verabreicht wird.

3. Zusammensetzung zur Verwendung gemäß Anspruch 2, wobei die Beschädigung aus Gewebezerstörung, Entzündung, Nekrose, Fibrose, chirurgischem Eingriff und Organversagen ausgewählt ist.

4. Zusammensetzung zur Verwendung gemäß mindestens einem der Ansprüche 1 bis 3, wobei die Geweberegeneration Differenzierung und/oder Proliferation von Gewebestammzellen involviert.

5. Zusammensetzung zur Verwendung gemäß mindestens einem der Ansprüche 1 bis 4, wobei die Geweberegeneration Proliferation von parenchymalen Gewebezellen umfasst.

6. Zusammensetzung zur Verwendung gemäß mindestens einem der Ansprüche 1 bis 5, wobei das Subjekt einen Zustand aufweist, in dem die Geweberegeneration unterdrückt ist, wobei bevorzugt der Zustand der unterdrückten Geweberegeneration aus der Gruppe ausgewählt ist, die aus Entzündung, Nekrose, Fibrose, Organversagen, verringerter Blutplättchenzahl, genetischen Anomalien und Reduktion von Noradrenalin besteht.

7. Zusammensetzung zur Verwendung bei der Differenzierung und/oder Proliferation von Leberstammzellen in einem Subjekt, wobei die Zusammensetzung ein Kollagen umfasst, das mit MMP14 behandelt wurde, wobei das Kollagen in einem Zustand ist, in dem RGD-Sequenz bereit ist, mit Zellen zu interagieren.

8. Verfahren zum Differenzieren und/oder Proliferieren von Leberstammzellen *in vitro,* welches einen Schritt des Inkontaktbringens eines mit MMP14 behandelten Kollagens mit den Leberstammzellen umfasst, wobei das Kollagen in einem Zustand ist, in dem RGD-Sequenz bereit ist, mit Zellen zu interagieren.

9. Zusammensetzung zur Verwendung bei der Förderung der Leberzellproliferation in einem Subjekt, wobei die Zusammensetzung ein mit MMP14 behandeltes Kollagen umfasst, wobei das Kollagen in einem Zustand ist, in dem RGD-Sequenz bereit ist, mit Zellen zu interagieren.

10. Verfahren zum Fördern der Leberzellproliferation *in vitro,* welches einen Schritt des Inkontaktbringens eines mit MMP14 behandelten Kollagens mit der Leberzelle umfasst, wobei das Kollagen in einem Zustand ist, in dem RGD-Sequenz bereit ist, mit Zellen zu interagieren.

## Revendications

1. Composition destinée à être utilisée pour favoriser la régénération tissulaire d'un foie chez un sujet, la composition comprenant un collagène qui a été traité avec MMP14, dans laquelle le collagène se trouve dans un état où une séquence RGD est prête à interagir avec des cellules.

2. Composition destinée à être utilisée selon la revendication 1, dans laquelle la régénération tissulaire se produit au niveau d'un tissu endommagé ou d'un tissu greffé, éventuellement la composition est administrée dans les quatre jours, de préférence le jour suivant le jour de l'endommagement subi ou de la greffe.

3. Composition destinée à être utilisée selon la revendication 2, dans laquelle l'endommagement est sélectionné parmi une destruction des tissus, une inflammation, une nécrose, une fibrose, une chirurgie invasive et une défaillance d'un organe.

4. Composition destinée à être utilisée selon l'une quelconque des revendications 1 à 3, dans laquelle la régénération tissulaire implique une différentiation et/ou une multiplication des cellules souches de tissu.

5. Composition destinée à être utilisée selon l'une quelconque des revendications 1 à 4, dans laquelle la régénération tissulaire implique une multiplication de cellules parenchymateuses de tissu.

6. Composition destinée à être utilisée selon l'une quelconques des revendications 1 à 5, dans laquelle le sujet est atteint d'une maladie dans laquelle la régénération tissulaire est supprimée, de préférence la maladie de régénération tissulaire supprimée est sélectionnée parmi le groupe constitué d'une inflammation, d'une nécrose, d'une fibrose, d'une défaillance d'un organe, d'une diminution du nombre de plaquettes, d'anomalies génétiques et d'une réduction de la noradrénaline.

7. Composition destinée à être utilisée dans la différentiation et/ou la multiplication de cellules souches hépatiques chez un sujet, la composition comprenant un collagène qui a été traité avec MMP14, dans laquelle le collagène se trouve dans un état où une séquence RGD est prête à interagir avec des cellules.

8. Procédé pour la différentiation et/ou la multiplication de cellules souches hépatiques in vitro, comprenant une étape de mise en contact d'un collagène qui a été traité avec MMP14 avec les cellules souches hépatiques, dans lequel le collagène se trouve dans un état où une séquence RGD est prête à interagir avec des cellules.

9. Composition destinée à être utilisée pour favoriser la multiplication de cellules hépatiques chez un sujet, la composition comprenant un collagène qui a été traité avec MMP14, dans laquelle le collagène se trouve dans un état où une séquence RGD est prête à interagir avec des cellules.

10. Procédé pour favoriser la multiplication de cellules hépatiques in vitro, comprenant une étape de mise en contact d'un collagène qui a été traité avec MMP14 avec la cellule hépatique, dans lequel le collagène se trouve dans un état où une séquence RGD est prête à interagir avec des cellules.


### SEQUENCE LISTING

<110> Nitto Denko Corporation
<120> Agent for promoting tissue regeneration
<130> PCT2629ND
<150> JP 2012-280261
   <151> 2012-12-21
<160> 9
<170> PatentIn version 3.5
<210> 1
   <211> 2063
   <212> DNA
   <213> Rattus norvegicus
<400> 1
   agcggacgct gtctgaggag tagagcctgc ctgagagcga gtgtcccgtc ggtcccggag      60
   cagcccagcc cggcccagcc ctcacaggtc ctctgtggtg cacacagccc acctcccagc     120
   catgcgctct ctccttctgg gcaccttatg cctcctggct gtggccctgg cagccgaggt     180
   gaagaaacct gtagaggccg cagcccctgg tactgcggag aagctgagtt ccaaggcgac     240
   cacactggca gagcgcagca caggcctggc cttcagcctg tatcaggcaa tggccaaaga     300
   tcaggcggtg gagaatatcc tcctgtcacc cttggtggtg gcctcatccc tgggccttgt     360
   gtcactgggt ggtaaagcca ccacagcttc gcaggcgaag gcagtgttga gcgctgagaa     420
   gctgcgggat gaggaggtgc acacggggct gggcgagctc gtgcgctccc tcagtaactc     480
   cactgcgcgc aatgtgacct ggaaactggg cagccgcctg tacgggccca gctccgtgag     540
   cttcgccgat gacttcgtgc gcagcagtaa gcaacactac aactgtgaac actccaagat     600
   caacttccga gacaagcgca gcgcgctgca gtccatcaac gagtgggcct cgcagaccac     660
   cgacggcaag ctgccagagg tcaccaagga tgtggagcgc acggatgggg cactgctcgt     720
   taatgccatg ttctttaagc cgcactggga tgagaagttc caccataaga tggtagacaa     780
   ccgtggcttc atggtgaccc gctcctacac tgtgggtgtt acgatgatgc accggacagg     840
   cctctacaac tactatgacg acgagaagga gaagctgcag ctggtggaga tgcccttggc     900
   ccataagctc tccagcctca tcatcctcat gccccaccac gtagaaccgc tcgagcgcct     960
   ggagaagttg ctgaccaagg agcaactaaa gacctggatg gggaagatgc agaagaaggc    1020
   tgttgccatc tccctgccca agggagtggt ggaggtgacc catgacctgc agaaacatct    1080
   ggccggactg ggcctgactg aggccatcga caagaacaag gcagacttat cgcgcatgtc    1140
   tggcaagaag gatctgtacc tggccagtgt gttccacgcc actgccttcg agtgggacac    1200
   agagggcaac ccctttgacc aggacatcta cgggcgtgag gagctgcgca gccccaagct    1260
   gttctatgcc gaccacccct tcatcttcct ggtgcgagat aatcagagcg gctccttgct 1320
   gttcattggc cgcctggtcc ggcccaaggg agacaagatg cgagacgagt tgtagagtcc 1380
   aagagtggtc gtggcgtggc atggcgggaa gtagccagag gttcctgaga cacatgggtg 1440
   ctattgtggg gggaggggga ggcaccaacc ctggatagtc cacgggtggg ggggggttgg 1500
   cagtgcagat cggaattccc ataggtctga gtaggtattt ttagacaaga gtccactccg 1560
   ttagacatgg agcccagaca ctatgagacc aaattcaggg gtgtcacagc cattctagcc 1620
   ctgccccaca agttttatat ccaatctagc ctcaacagtc aatcagtgtt catattcgtg 1680
   gccaagcatt ctgtctgtga gacaatcgag ttgggtgggg gtagggcggc ctagcctctc 1740
   tgtcaccatg ccctgagccc tcggttttcc tgttcacctc tgattccctt taaccacaaa 1800
   actaggagcc tgtggtccca gaactgccca cccttaaaat cgattctttg ccgcagcagt 1860
   gggagttggg gagaccatgc ataggagggc tccttggcat atccggccct agagccatta 1920
   cattacattg acaactcaac tttgcttctg ttttctccct ttttgagttt ttcaaggaat 1980
   gggggtttgg ggaaggggaa gcctttattg ctttcaaaac aagaacagtt tgtcaatttt 2040
   ttttgaataa accttttcca atg 2063
<210> 2
   <211> 27
   <212> RNA
   <213> Artificial sequence
<220>
   <223> gp46 siRNA sense strand
<400> 2
   guuccaccau aagaugguag acaacag 27
<210> 3
   <211> 27
   <212> RNA
   <213> Artificial sequence
<220>
   <223> gp46 siRNA antisense strand
<400> 3
   guugucuacc aucuuauggu ggaacau 27
<210> 4
   <211> 27
   <212> RNA
   <213> Artificial sequence
<220>
   <223> siRNA random sense strand
<400> 4
   cgauucgcua gaccggcuuc auugcag 27
<210> 5
   <211> 27
   <212> RNA
   <213> Artificial sequence
<220>
   <223> siRNA random antisense strand
<400> 5
   gcaaugaagc cggucuagcg aaucgau 27
<210> 6
   <211> 21
   <212> DNA
   <213> Artificial sequence
<220>
   <223> MMP14 siRNA sense strand
<400> 6
   gcucauucau ggguagcgat t 21
<210> 7
   <211> 21
   <212> DNA
   <213> Artificial sequence
<220>
   <223> MMP14 siRNA antisense strand
<400> 7
   ucgcuaccca ugaaugagcc t 21
<210> 8
   <211> 25
   <212> RNA
   <213> Artificial sequence
<220>
   <223> HGF siRNA sense strand
<400> 8
   auaucuuucc ggcaagaauu ugugc 25
<210> 9
   <211> 25
   <212> RNA
   <213> Artificial sequence
<220>
   <223> HGF siRNA antisense strand
<400> 9
   gcacaaauuc uugccggaaa gauau 25